# EUROPÄISCHE PATENTANMELDUNG

(11) **EP 2 039 293 A1**
(43) Veröffentlichungstag der Anmeldung: **25.03.2009**
(21) Anmeldenummer: 07116748.0
(22) Anmeldetag: 19.09.2007
(51) Int. Cl.: A61B 5/15

(54) **Kombinationsantrieb für ein Probengewinnungssystem zum Gewinnen einer flüssigen Probe**

(71) Anmelder: F. Hoffmann-La Roche AG, 4070 Basel (CH); Roche Diagnostics GmbH, 68305 Mannheim (DE)
(72) Erfinder: Konya, Ahmet, 67165 Waldsee (DE); Harttig, Herbert, Dr., 67434 Neustadt (DE)
(74) Vertreter: Huhn, Michael

(57) **Zusammenfassung**

Es wird ein Probengewinnungssystem (110) zum Gewinnen einer flüssigen Probe vorgeschlagen, welches mindestens ein analytisches Hilfsmittel (112) aufweist. Das Probengewinnungssystem (110) weist ein Kopplungselement (152) zur Ankopplung an das analytische Hilfsmittel (112) auf sowie mindestens eine Antriebseinheit (160) zum Antreiben einer Bewegung des Kopplungselements (152) aus einer Ruheposition in eine ausgelenkte Position. Die Antriebseinheit (160) umfasst einen Energiewandler (162), welcher ausgestaltet ist, um eine Drehbewegung mit unterschiedlichen Drehrichtungen zu erzeugen. Die Antriebseinheit (160) weist weiterhin eine Kopplungsvorrichtung mit mindestens einem drehrichtungssensitiven Element (194, 218; 242) auf, wobei die Kopplungsvorrichtung eingerichtet ist, um in einer ersten Drehrichtung den Energiewandler (162) an eine erste Systemfunktion und in einer zweiten, von der ersten Drehrichtung verschiedenen Drehrichtung an eine zweite, von der ersten Systemfunktion verschiedene Systemfunktion anzukoppeln.

## Beschreibung

### Gebiet der Erfindung

Die Erfindung betrifft ein Probengewinnungssystem zum Gewinnen einer flüssigen Probe mittels mindestens eines analytischen Hilfsmittels. Die Erfindung betrifft weiterhin ein Verfahren zum Gewinnen einer flüssigen Probe. Derartige Probengewinnungssysteme und Verfahren werden insbesondere in der medizinischen Diagnostik im Krankenhausbereich, im Bereich von Pflegeheimen oder im Rahmen eines Home-Monitoring Konzeptes eingesetzt, um die Konzentration mindestens eines Analyten, beispielsweise eines Metaboliten und insbesondere von Blutglucose, in einer Körperflüssigkeit zu bestimmen.

### Stand der Technik

Die Untersuchung von Blutproben oder anderen Proben von Körperflüssigkeit, wie beispielsweise von interstitieller Flüssigkeit, ermöglichen in der klinischen Diagnostik das frühzeitige und zuverlässige Erkennen von pathologischen Zuständen sowie die gezielte und fundierte Kontrolle von Körperzuständen. Die medizinische Diagnostik setzt in der Regel die Gewinnung einer Probe aus Blut oder interstitieller Flüssigkeit des zu untersuchenden Individuums voraus.

Zur Gewinnung der Probe kann die Haut z. B. an der Fingerbeere oder dem Ohrläppchen der zu untersuchenden Person mit Hilfe einer sterilen, spitzen oder scharfen Lanzette perforiert werden, um so einige wenige Mikroliter Blut oder weniger für die Analyse zu gewinnen. Insbesondere eignet sich diese Methode für die Analyse einer Probe, die unmittelbar nach der Probengewinnung durchgeführt wird.

Vor allem im Bereich des so genannten "Home-Monitoring", also dort, wo medizinische Laien selbst einfache Analysen des Bluts oder der interstitiellen Flüssigkeit durchführen, und dort insbesondere für die regelmäßige, mehrmals täglich durchzuführende Blutgewinnung durch Diabetiker für die Kontrolle der Blutglukosekonzentration, werden Lanzetten und dazu passende Geräte (so genannte Stechhilfen) angeboten, die eine möglichst schmerzarme und reproduzierbare Probengewinnung ermöglichen. Solche Lanzetten und Geräte (Stechhilfen) sind z. B. Gegenstand von WO-A 98/48695, US 4,442,836, US 5,554,166 oder WO 2006/013045 A1.

Die eigenhändige Blutzuckerbestimmung ist heute eine weltweit verbreitete Methode in der Diabetes-Kontrolle. Blutzuckergeräte im Stand der Technik weisen in der Regel ein Analysegerät auf, in das ein Testelement (Teststreifen) eingeführt wird. Das Testelement wird mit einem Tropfen einer Probe in Kontakt gebracht, der zuvor mittels einer Stechhilfe z. B. aus der Fingerbeere gewonnen wurde.

Zur Analyse von Flüssigproben, beispielsweise Körperflüssigkeiten wie Blut oder Urin, werden häufig Analysegeräte verwendet, bei denen sich die zu analysierende Probe auf einem Testfeld eines Testelements befindet und in dem Testfeld gegebenenfalls mit einer oder mehreren Reagenzien reagiert, bevor sie analysiert wird. Die optische, insbesondere photometrische, und die elektrochemische Auswertung von Testelementen stellen die gebräuchlichsten Verfahren zur schnellen Bestimmung der Konzentration von Analyten in Proben dar. Analysesysteme mit Testelementen zur Probenanalyse werden allgemein im Bereich der Analytik, der Umweltanalytik und vor allem im Bereich der medizinischen Diagnostik eingesetzt. Insbesondere im Bereich der Blutglukosediagnostik aus Kapillarblut besitzen Testelemente, die photometrisch oder elektrochemisch ausgewertet werden, einen großen Stellenwert.

Es gibt verschiedene Formen von Testelementen. Bekannt sind zum Beispiel im Wesentlichen quadratische Plättchen, die auch als Slides bezeichnet werden, in deren Mitte sich ein mehrschichtiges Testfeld befindet. Diagnostische Testelemente, die streifenförmig ausgebildet sind, werden als Teststreifen bezeichnet. Im Stand der Technik sind Testelemente umfassend beschrieben, beispielsweise in den Dokumenten CA 2311496 A1, US 5,846,837 A, US 6,036,919 A oder WO 97/02487.

Weitere im Stand der Technik bekannte mehrschichtige Testelemente sind Analysebänder mit einer Vielzahl von Testfeldem, die in einer Kassette aufgewickelt zur Verwendung in einem Analysegerät bereitgestellt werden. Solche Kassetten und Analysebänder werden zum Beispiel in den Dokumenten DE 103 32 488 A1, DE 103 43 896 A1, EP 1 424 040 A1, WO 2004/056269 A1 und US 2006/0002816 A1 beschrieben.

Die zahlreichen Systemkomponenten (Lanzette, Stechhilfe, Testelement und Analysegerät) benötigen viel Platz und bedingen eine relativ komplexe Handhabung. Inzwischen gibt es auch Systeme mit einem höheren Grad der Integration und damit einer einfacheren Handhabung, bei denen z. B. die Testelemente im Analysegerät magaziniert und für die Messung zur Verfügung gestellt werden. Ein nächster Schritt der Miniaturisierung ist beispielsweise durch die Integration von mehreren Funktionen beziehungsweise Funktionselementen in einem einzigen analytischen Hilfsmittel (Disposable) zu erreichen. Durch geeignete Kombination von Stechvorgang und sensorischer Analytkonzentrations-Erfassung auf einem Testelement lässt sich beispielsweise der Bedienablauf deutlich vereinfachen.

Aus US 2006/0155317 A1 ist eine Lanzettenvorrichtung zum Erzeugen einer Einstichwunde in einer Hautoberfläche bekannt, welche ein integriertes Testelement in Form eines Referenzelements mit einer Lanzette und einer Probenaufnahmeeinheit umfasst. Das Testelement wird einmal fest an einen Kopplungsmechanismus der Lanzettenvorrichtung angekoppelt. In einer ersten Stellung des Kopplungsmechanismus wird mittels einer Kopplungsstange und einer Pleuelstange die Lanzette des Testelements betätigt und eine Stechbewegung durchgeführt. Anschließend wird der gesamte Kopplungsmechanismus mitsamt dem darin fest angekoppelten Testelement durch eine Schwenkbewegung in eine zweite Position bewegt, in welcher eine Öffnung eines Probenaufnahmekanals des Testelements über der Einstichstelle liegt, um eine flüssige Probe aufzunehmen.

Aus WO 2005/107596 A2 ist das Bereitstellen einer Vielzahl voneinander beabstandeter Lanzetten auf einem Band bekannt. Gemäß einer Ausführungsvariante trägt das Band nicht nur die Lanzetten, sondern auch eine Vielzahl von Testelementen, die jeweils einer der Lanzetten zugeordnet sind. Dabei handelt es sich daher um ein Band mit einer Vielzahl von zueinander beabstandet angeordneten analytischen Hilfsmitteln, die eine Integration von Stechvorgängen und Probenaufnahmevorgängen in einem Probengewinnungssystem ermöglichen.

Für integrierte Messsysteme spielt die Baugröße eine besondere Rolle. Ein Ziel integrierter Systeme muss es sein, ein Gerät bereitzustellen, das nicht viel größer ist als die bisherigen, nicht-integrierten Systeme. Ein Ansatz in dieser Richtung besteht darin, Kombinationsantriebe zu verwenden, bei welchen ein Bewegungsablauf mehrere Funktionen übernimmt. So ist beispielsweise aus WO 2006/013045 ein System bekannt, bei welchem ein elektrisch betriebener Motor zum einen Energie für einen mechanischen Energiespeicher bereitstellt, und zum anderen jedoch auch gleichzeitig oder zeitlich unabhängig davon eine weitere Systemfunktion betreibt. Diese Systemfunktion kann beispielsweise ein Magazintransport oder ein Testelementtransport sein. Um den Motor zeitlich nacheinander für verschiedene Funktionen zu verwenden, wird ein Getriebe und/oder eine Kupplung vorgeschlagen, die den Motor aktiv an die entsprechende Systemfunktion, welche gerade gewünscht wird, ankoppelt oder wieder von dieser entkoppelt. Verschiedene Kupplungssysteme werden vorgeschlagen.

Die in WO 2006/013045 vorgeschlagene Vorrichtung stellt somit einen wesentlichen Schritt hin zu einem höheren Integrationsgrad dar. Dabei sind jedoch noch nicht alle Integrationsmöglichkeiten ausgeschöpft, da auch in dem in WO 2006/013045 vorgeschlagenen System nach wie vor weitere Systemfunktionen anfallen, welche in der Regel mit separaten Antrieben mit Energie versorgt werden müssen. Weiterhin sind die aktiven Kupplungselemente, welche in WO 2006/013045 vorgeschlagen werden und den Antrieb an verschiedene Systemfunktionen koppeln, technisch aufwändig und unter Umständen anfällig gegenüber Störungen.

### Aufgabe der Erfindung

Es ist daher eine Aufgabe der vorliegenden Erfindung, ein Probengewinnungssystem, insbesondere ein Probengewinnungssystem gemäß der obigen Beschreibung, bereitzustellen, welches zum Gewinnen einer flüssigen Probe, insbesondere einer Körperflüssigkeit, geeignet ist und welches die Nachteile der bekannten Systeme vermeidet. Insbesondere soll das Probengewinnungssystem einen hohen Integrationsgrad aufweisen und einen einfachen und störungsunanfälligen Kombinationsantrieb für verschiedene Systemfunktionen des Probengewinnungssystems bereitstellen.

### Beschreibung der Erfindung

Diese Aufgabe wird gelöst mittels eines Probengewinnungssystems mit den Merkmalen des Anspruchs 1. Vorteilhafte Weiterbildungen der Erfindung, welche einzeln und in Kombination realisiert werden können, sind in den abhängigen Ansprüchen dargestellt. Der Wortlaut sämtlicher Ansprüche wird hiermit zum Inhalt der Beschreibung gemacht.

Das vorgeschlagene Probengewinnungssystem soll eingerichtet sein, um insbesondere für einen Analytnachweis, wie beispielsweise einen qualitativen und/oder quantitativen Nachweis von Blutglukose, eine flüssige Probe, insbesondere eine Körperflüssigkeit, zu gewinnen. Dabei ist unter einem "Probengewinnungssystem" ein System zu verstehen, welches eingerichtet ist, um diese flüssige Probe zu erzeugen und/oder die flüssige Probe aufzunehmen und/oder die aufgenommene Probe zumindest teilweise bereits zu verarbeiten, das heißt ganz oder teilweise zu analysieren. Der Begriffsinhalt des Begriffs "Probengewinnungssystem" ist insofern weit zu fassen.

Das Probengewinnungssystem umfasst mindestens ein analytisches Hilfsmittel. Dieses analytische Hilfsmittel kann beispielsweise die Probenaufnahme, die Probenerzeugung, die Analyse der Probe, eine Kombination dieser Funktionen oder ähnliche Aufgaben übernehmen. Besonders bevorzugt ist es dabei, wenn das analytische Hilfsmittel mindestens eine Lanzette und/oder mindestens ein Testelement mit mindestens einem Testfeld zur Analyse der flüssigen Probe aufweist. Beispielsweise kann es sich bei der Lanzette um eine Lanzettennadel oder um eine Lanzette mit einem scharfkantigen Element handeln, welches beispielsweise für die Perforation einer Hautpartie im Bereich der Fingerbeere und/oder des Ohrläppchens geeignet ist. Das Testelement kann beispielsweise gemäß einem der oben beschriebenen Testelemente ausgestaltet sein und kann beispielsweise ein Testfeld für einen optischen Analytnachweis und/oder für einen elektrochemischen Analytnachweis, jeweils mittels einer geeigneten Testchemie, umfassen.

Besonders bevorzugt ist es dabei, wenn mehrere analytische Hilfsmittel vorgesehen sind oder ein analytisches Hilfsmittel, welches eine Mehrzahl von Untereinheiten analytischer Hilfsmittel (beispielsweise eine Mehrzahl von Lanzetten und/oder eine Mehrzahl von Testelementen) umfasst. Beispielsweise kann das Probengewinnungssystem zur Aufnahme und Bereitstellung mehrerer analytischer Testelemente geeignete Elemente umfassen, beispielsweise ein Magazin, insbesondere ein Trommelmagazin, ein Stangenmagazin, ein Reihenmagazin oder Zickzackmagazin, ein Analyseband mit einer Vielzahl von analytischen Hilfsmitteln, eine Analysescheibe, auf welcher mehrere analytische Hilfsmittel angeordnet sind oder ähnliche Elemente, welche die Bereitstellung und Verwendung einer Vielzahl dieser analytischen Hilfsmittel ermöglichen. Besonders bevorzugt ist es im Rahmen der vorliegenden Erfindung, wenn das analytische Hilfsmittel ein Analyseband aufweist, wobei auf dem Analyseband eine Vielzahl von Lanzetten und/oder Testfeldern angeordnet sind. Dabei ist es wiederum bevorzugt, wenn Lanzetten und Testfelder alternierend angeordnet sind, so dass beispielsweise jeweils einer Lanzette ein nachgeordnetes Testfeld zugeordnet ist. Auch Bänder, welche ausschließlich Lanzetten aufweisen, sowie Bänder, welche ausschließlich Testfelder aufweisen, sind im Rahmen der Erfindung anwendbar. Analysebänder mit hierauf angeordneten Lanzetten sind auch als "lancet on tape"-(LOT-)Analysebänder bekannt. Dabei ist der Begriff "Band" im Zusammenhang mit "Analyseband" vorzugsweise dahingehend zu verstehen, dass dieses Analyseband einen kontinuierlichen Trägerstreifen umfasst, beispielsweise einen Trägerstreifen aus einem Polymermaterial (zum Beispiel eine Polyesterfolie), einem Papiermaterial oder einem Verbundmaterial. Der Begriff "Band" ist jedoch nicht auf durchgehende Bänder beschränkt, sondern umfasst prinzipiell jegliche serielle Verbindungstechnik benachbarter analytischer Hilfsmittel, wie beispielsweise Lanzetten und/oder Testelemente, welche eine serielle Zufuhr dieser analytischen Hilfsmittel in eine Applikationsposition beinhaltet. So sind beispielsweise Gliederketten, Haken- und Ösenverbindungen, Verbindungen über Zwischenglieder oder ähnliche Arten von seriellen Verbindungen zwischen benachbarten analytischen Hilfsmitteln von diesem Begriff mit umfasst. Es sei weiterhin darauf hingewiesen, dass die Erfindung jedoch nicht auf die Verwendung von Analysebändern beschränkt ist, sondern dass diese beispielsweise auch für andere Methoden der Bereitstellung von analytischen Hilfsmitteln eingesetzt werden kann.

Das Probengewinnungssystem umfasst ein Kopplungselement zur Ankopplung an das analytische Hilfsmittel, sowie mindestens eine Antriebseinheit zum Antreiben einer Bewegung des Kopplungselements aus einer Ruheposition in eine ausgelenkte Position. Die Ankopplung erfolgt dabei vorzugsweise derart, dass nach der Verwendung des analytischen Hilfsmittels (beispielsweise einer Lanzette) wiederum eine Abkopplung von diesem erfolgt, so dass anschließend erneut an ein nächstes analytisches Hilfsmittel angekoppelt werden kann, beispielsweise ein Testelement, beispielsweise ein auf demselben Analyseband wie die zuvor verwendete Lanzette angeordnetes Testelement.

Unter einer Kopplung oder einem Ankoppeln kann dabei insbesondere eine Herstellung eines physikalischen Kontaktes verstanden werden, und unter einer Abkopplung die Auf gabe oder Trennung dieses Kontaktes. Das Kopplungselement kann aktiv und/oder passiv an das analytische Hilfsmittel in der Applikationsposition ankoppeln und kann hierdurch bewirken, dass dieses analytische Hilfsmittel ebenfalls eine Bewegung um einen definierten Weg in eine oder mehrere ausgelenkte Positionen durchführt. Dabei können für unterschiedliche Arten analytischer Hilfsmittel unterschiedliche ausgelenkte Positionen vorgesehen sein, beispielsweise eine ausgelenkte Position für eine Probenaufnahme mittels eines Testelements und eine ausgelenkte Position für eine Stechbewegung einer Lanzette.

Unter einer "aktiven" Kopplung können dabei verschiedene Arten der Kopplung des Kopplungselements mit dem analytischen Hilfsmittel zu verstehen sein. So kann beispielsweise das Kopplungselement das analytische Hilfsmittel aktiv greifen, beispielsweise mittels eines Greifers und/oder eines auf andere Weise gestalteten Öffnungs- oder Schließmechanismus, welcher ein aktives An- und Abkoppeln erlaubt. Im Gegensatz hierzu wird in diesem Sinne unter einer passiven Kopplung eine Kopplung ohne aktives Öffnen oder Schließen verstanden, also beispielsweise eine einfache Ausübung einer Kraft durch das Kopplungselement auf das analytische Hilfsmittel oder ein Ankoppeln mittels Widerhaken.

Auch in einem anderen Sinne kann eine aktive oder eine passive Kopplung verstanden werden, wobei wiederum beide Möglichkeiten realisierbar sind, nämlich im Sinne eines aktiven oder passiven Antriebs des analytischen Hilfsmittels durch das Kopplungselement. Unter einer aktiven Kopplung kann dabei eine Kopplung verstanden werden, bei welcher das Kopplungselement an das analytische Hilfsmittel derart ankoppelt (zum Beispiel durch eine kraftschlüssige und/oder formschlüssige Kopplung, beispielsweise durch ein Greifen der Lanzette oder des Testelements oder durch eine Mikrostruktur mit Widerhaken, welche beim Zurückziehen des Kopplungselements auch die Lanzette oder das Testelement mit zurückziehen), so dass auch die Rückbewegung der Lanzette oder des Testelements aus der ausgelenkten Position in die Ruheposition durch das Kopplungselement geführt und angetrieben wird. Bei einer passiven Kopplung hingegen schiebt oder stößt das Kopplungselement das analytische Hilfsmittel, beispielsweise die Lanzette oder das Testelement, in die ausgelenkte Position. Die Rückbewegung des analytischen Hilfsmittels in die Ruheposition muss dann durch ein zusätzliches Antriebselement erfolgen, beispielsweise durch eine Feder, die bei der Auslenkung der Lanzette oder des Testelements gespannt wird und bei ihrer Entspannung auf die Lanzette oder das Testelement wirkt, so dass eine Rückbewegung der Lanzette oder des Testelements in die Ruheposition erfolgt.

Die Ankopplung des Kopplungselements an das analytische Hilfsmittel kann auf verschiedene Weisen erfolgen, welche beispielsweise an die Ausgestaltung des analytischen Hilfsmittels angepasst sein können. Dabei kann ein grundsätzlich ein beliebiger Antrieb verwendet werden, welcher geeignet ist, einen definierten Hub des analytischen Hilfsmittels zu bewirken. Als Beispiele können dabei Exzenterantriebe, Kniegelenkantriebe oder ähnliche Antriebe genannt werden. Im Rahmen der vorliegenden Erfindung ist es jedoch besonders bevorzugt, insbesondere wenn ein Analyseband verwendet wird, wenn das Kopplungselement einen Pleuelantrieb und/oder einen Kulissenantrieb umfasst. Der Pleuelantrieb kann beispielsweise eine Pleuelstange umfassen. Der Pleuelantrieb und/oder der Kulissenantrieb sollen derart ausgestaltet sein, dass diese an ein in einer Applikationsposition angeordnetes analytisches Hilfsmittel ankoppeln. Unter einer "Applikationsposition" ist dabei im Rahmen der vorliegenden Erfindung eine Position zu verstehen, in welcher das analytische Hilfsmittel für eine Probengewinnungsfunktion des Probengewinnungssystems einsetzbar ist. Beispielsweise kann es sich dabei um eine Position handeln, in welcher eine Lanzette eine Stechbewegung zur Perforation einer Hautpartie durchführt und/oder eine Position, in welcher ein Testelement für das Sammeln bzw. Aufnehmen einer flüssigen Probe eingesetzt wird. In beiden Fällen wird das analytische Hilfsmittel (das heißt im einen Fall die Lanzette und in anderem Fall das Testelement) aus seiner Ruheposition ausgelenkt, um die beschriebene Funktion wahrzunehmen. Sind unterschiedliche Arten analytischer Hilfsmittel vorgesehen, wie beispielsweise eine Lanzette und ein Testelement, so können auch mehrere Applikationspositionen für diese unterschiedlichen Arten analytischer Hilfsmittel vorgesehen sein. Vorzugsweise wird jedoch für alle analytischen Hilfsmittel ein und dieselbe Applikationsposition verwendet. Beispielsweise kann die Applikationsposition eine Öffnung in einem Gehäuse des Probengewinnungssystems umfassen, beispielsweise eine durch eine Klappe oder einen Schieber verschließbare Öffnung.

Die Antriebseinheit, welche die Bewegung des Kopplungselements antreibt, umfasst einen Energiewandler. Dieser Energiewandler ist vorzugsweise als elektromechanischer Energiewandler ausgestaltet. Unter einem "elektromechanischen Energiewandler" ist dabei im Rahmen der vorliegenden Erfindung ein Wandler zu verstehen, welcher eingerichtet ist, um elektrische Energie, beispielsweise über eine elektrische Energieversorgung (zum Beispiel ein Stromkabel) und/oder einen elektrischen Energiespeicher (zum Beispiel einen Akkumulator, eine Batterie oder eine Kapazität) bereitgestellte elektrische Energie in mechanische Energie umzuwandeln. Dabei ist im Rahmen der vorliegenden Erfindung besonders die Verwendung eines Elektromotors bevorzugt. Alternativ oder zusätzlich kann der Energiewandler jedoch auch andere Arten von elektromechanischen Energiewandlern umfassen, beispielsweise Aktuatoren, Pumpen oder ähnliche Wandler oder Wandlerkombinationen. Prinzipiell wäre anstelle des bevorzugt eingesetzten elektrisch-mechanischen (d.h. elektromechanischen) Energiewandlers jedoch auch eine beliebige andere Art eines Energiewandlers denkbar, also ein Wandler, welcher eingerichtet ist, um eine Energieform in eine mechanische Energie, vorzugsweise eine Rotationsenergie, umzuwandeln. Beispielsweise wäre ein mechanisch betätigter Antrieb, also ein mechanisch-mechanischer Energiewandler möglich. Insofern ist der Begriff des Energiewandlers weit zu fassen und soll grundsätzlich jede Art des Antriebs umfassen, bei welcher eine Energieform (beispielsweise eine elektrische und/oder mechanische Energie) in eine Bewegungsenergie umgewandelt wird.

Der Energiewandler ist ausgestaltet, um eine Drehbewegung zu erzeugen, welche unterschiedliche Drehrichtungen beinhaltet. Bei der Verwendung eines Elektromotors kann dies beispielsweise durch einfaches Umpolen erfolgen, was zu einer Umkehr der Drehrichtung führt.

Eine Grundidee der vorliegenden Erfindung liegt darin, den Integrationsgrad des Probengewinnungssystems dadurch zu erhöhen, dass diese unterschiedlichen Drehrichtungen des (vorzugsweise elektromechanischen) Energiewandlers für unterschiedliche Systemfunktionen genutzt werden. Zu diesem Zweck umfasst die Antriebseinheit weiterhin eine Kopplungsvorrichtung, welche an den Energiewandler ankoppeln kann und von diesem bereitgestellte mechanische Energie, insbesondere Rotationsenergie, unterschiedlichen Systemfunktionen zuführen kann. Zu diesem Zweck weist die Kopplungsvorrichtung mindestens ein drehrichtungssensitives Element auf und ist eingerichtet, um in einer ersten Drehrichtung den Energiewandler an mindestens eine erste Systemfunktion und in einer zweiten, von der ersten Drehrichtung verschiedenen Drehrichtung an mindestens eine zweite, von der ersten Systemfunktion verschiedene Systemfunktion anzukoppeln.

Unter einem An- bzw. Abkoppeln einer Systemfunktion ist dabei eine Aktion des Probengewinnungssystems zu verstehen, bei welcher die jeweilige Systemfunktion aktiviert und verwendet bzw. deaktiviert und nicht mehr verwendet wird. Bei der ersten und/oder der zweiten Systemfunktion kann es sich dabei um eine Vielzahl möglicher Funktionen des Probengewinnungssystems oder eine Kombination derartiger Funktionen handeln. Besonders bevorzugt ist es dabei, wenn die erste Systemfunktion und/oder die zweite Systemfunktion mindestens eine der folgenden Funktionen des Probengewinnungssystems umfassen: einen Antrieb einer Stechbewegung einer Lanzette des analytischen Hilfsmittels, eine Probennahmebewegung eines Testelements des analytischen Hilfsmittels, ein Spannen eines Energiespeichers zum Antreiben der Stechbewegung einer Lanzette, insbesondere eines mechanischen Energiespeichers (zum Beispiel eines Federelements), eine Transportfunktion eines analytischen Hilfsmittels zum Bereitstellen des analytischen Hilfsmittels in einer Applikationsposition, eine Transportfunktion eines analytischen Hilfsmittels zum Bereitstellen des analytischen Hilfsmittels (insbesondere eines Testelements des analytischen Hilfsmittels) in einer Messposition, insbesondere einer Messposition zur optischen und/oder elektrochemischen Auswertung des Testelements, eine Transportfunktion eines Magazins des Probengewinnungssystems zum Bereitstellen eines analytischen Hilfsmittels aus einem Magazin in einer Applikationsposition (zum Beispiel ein Weitertakten eines Magazins), eine Transportfunktion eines mehrere analytische Hilfsmittel enthaltenden Analysebandes zum Bereitstellen eines analytischen Hilfsmittels in einer Applikationsposition (zum Beispiel ein Weitertakten des Analysebandes zum Bereitstellen eines Testelements und/oder ein Weitertakten zum Bereitstellen einer Lanzette), eine Transportfunktion einer mehrere analytische Hilfsmittel enthaltenden Analysescheibe zum Bereitstellen eines analytischen Hilfsmittels in einer Applikationsposition. Alternativ oder zusätzlich sind jedoch auch weitere Systemfunktionen einsetzbar. Dabei kann auch ein Zyklus verwendet werden, bei welchem im Rahmen eines einzelnen Zyklus in verschiedenen Phasen mehrfach in die Drehrichtungen gedreht wird, wobei jeweils ganz oder teilweise unterschiedliche Systemfunktionen angekoppelt werden. Beispielsweise kann ein Zyklus vier Phasen umfassen:
- erste Phase: Drehung in die erste Drehrichtung, Ankopplung Systemfunktion A,
- zweite Phase: Drehung in die zweite Drehrichtung, Ankopplung Systemfunktion B,
- dritte Phase: Drehung in die erste Drehrichtung, Ankopplung Systemfunktion C, und
- vierte Phase: Drehung in die zweite Drehrichtung, Ankopplung Systemfunktion D.

Auch andere Ausgestaltungen sind jedoch denkbar, beispielsweise Zyklen mit mehr oder weniger als vier Phasen und/oder die Ankopplung mehrerer Systemfunktionen in einer Phase und/oder die Wiederholung der Ankopplung einer Systemfunktion in verschiedenen Phasen (also beispielsweise Identität der Systemfunktionen A und C im obigen Beispiel).

Die mindestens eine erste Systemfunktion und die mindestens eine zweite Systemfunktion können dabei jeweils eine oder mehrere Systemfunktionen umfassen. Sind mehrere erste und/oder zweite Systemfunktionen vorgesehen, so können diese beispielsweise zeitgleich, zeitlich überlappend oder auch zeitlich versetzt durchgeführt werden. Dies kann beispielsweise dadurch erfolgen, dass in derselben Drehrichtung des Energiewandlers Systemfunktionen in unterschiedlichen Winkelstellungen angekoppelt werden, was, wie unten anhand von Beispielen näher erläutert wird, beispielsweise durch teilbezahnte Zahnräder oder ähnliche Kopplungsmechanismen bewirkt werden kann. Auch auf andere Weise kann ein zeitlicher Versatz der Ankopplung mehrerer Systemfunktionen bei derselben Drehrichtung erfolgen. Ein wichtiges Beispiel einer zeitversetzten Ankopplung mehrerer Systemfunktionen an eine Drehung in derselben Richtung ist eine Spannung bzw. Aufladung eines Energiespeichers, gefolgt von einem Auslösevorgang. Weitere Beispiele werden unten näher erläutert.

Das vorgeschlagene Probengewinnungssystem, bei welchem zwei verschiedene Drehrichtungen des Energiewandlers eingesetzt werden, um unterschiedliche Systemfunktionen zu aktivieren bzw. mit Energie zu versorgen, bietet insbesondere hinsichtlich der Systemkomplexität und der Zuverlässigkeit des Systems zahlreiche Vorteile gegenüber herkömmlichen integrierten Probengewinnungssystemen, beispielsweise den oben beschriebenen, aus dem Stand der Technik bekannten Systemen. So wird insbesondere der Integrationsgrad durch die Nutzung beider Drehrichtungen stark erhöht. Zudem ist ein aktives An- und Abkoppeln einzelner Systemfunktionen, wie dies beispielsweise durch die in WO 2006/013045 beschriebenen Kopplungsmechanismen erfolgt, nicht zwingend erforderlich. Somit kann auf eine unabhängige Kopplungsbewegung zum An- oder Abkoppeln der Systemfunktionen, welche einen zusätzlichen Aktuator, einen Benutzereingriff oder ähnliches erfordert, verzichtet werden. Die unterschiedlichen Systemfunktionen können beispielsweise ausschließlich durch die Drehrichtung eines Elektromotors oder eines anders gestalteten Energiewandlers aktiviert bzw. angekoppelt werden, was auf einfache Weise zum Beispiel durch eine elektronische Steuerung des Probengewinnungssystems realisierbar ist. Beispielsweise kann zu diesem Zweck das Probengewinnungssystem eine Steuerung umfassen, zum Beispiel einen Mikrocomputer und/oder eine andere Art von elektronischer Steuerung, welche die unterschiedlichen Systemfunktionen ankoppelt, indem die Drehrichtung des Energiewandlers vorgegeben wird. Auf diese Weise können einzelne Phasen oder Taktzyklen des Betriebs des Probengewinnungssystems auf einfache Weise gesteuert werden, ohne dass ein zusätzliches Betätigen von Kopplungselementen erforderlich wäre.

Das Probengewinnungssystem kann auf verschiedene Weise vorteilhaft weitergebildet werden, wobei die Weiterbildungen insbesondere von der Funktionalität des Probengewinnungssystems abhängig sein können. Umfasst das Probengewinnungssystem beispielsweise eine Lanzette zum Perforieren einer Hautpartie, so ist es besonders bevorzugt, wenn das Probengewinnungssystem weiterhin einen mechanischen Energiespeicher umfasst. Dieser mechanische Energiespeicher soll eingerichtet sein, um Energie für eine Stechbewegung einer Lanzette des analytischen Hilfsmittels abzugeben, welche wiederum über das Kopplungselement an die Lanzette übertragen wird. Diese Kopplung ist vorzugsweise derart ausgestaltet, dass die Lanzette bei der Stechbewegung eine Geschwindigkeit von ca. 2 bis 3 m/s erreicht, so dass als mechanischer Energiespeicher vorzugsweise ein Energiespeicher genutzt wird, welcher in der Lage ist, die gespeicherte Energie schnell abzugeben. Zu diesem Zweck haben sich insbesondere Energiespeicher als vorteilhaft erwiesen, welche ein Federelement umfassen, beispielsweise eine Schraubenfeder, eine Spiralfeder, eine Blattfeder, eine Tellerfeder oder eine andere Art von Federelement. Weiterhin kann, alternativ oder zusätzlich, der mechanische Energiespeicher jedoch auch ein elastisches Element umfassen, beispielsweise ein Elastomerelement (zum Beispiel ein Gummielement), welches durch Zusammendrücken und/oder Dehnen gespannt werden kann, so dass mechanische Energie gespeichert wird. Auch andere Arten von Energiespeichern sind jedoch einsetzbar, beispielsweise pneumatische Druckspeicher oder andere Arten von Speichern.

Wird ein derartiger mechanischer Energiespeicher verwendet, so sollte das Probengewinnungssystem insbesondere eine Vorrichtung aufweisen, welche eine schlagartige Freigabe der gespeicherten mechanischen Energie und eine Übertragung an die Lanzette ermöglicht. Hierbei können grundsätzlich eine Vielzahl von Auslösemechanismen eingesetzt werden, beispielsweise Auslösemechanismen, welche einen mechanischen Schalter zur Freigabe umfassen. Beispielsweise kann dieser Schalter durch eine Benutzeraktion aktiviert werden oder, alternativ oder zusätzlich, wiederum über eine elektronische Steuerung des Probengewinnungssystems. Im Rahmen der vorliegenden Erfindung ist es jedoch besonders bevorzugt, wenn auch das Auslösen der Lanzettenbewegung, also eine Freigabe der Energie des mechanischen Energiespeichers an die Lanzette, über die Antriebseinheit erfolgt, insbesondere über eine Drehbewegung des Energiewandlers. Zu diesem Zweck kann die Antriebseinheit beispielsweise ein Zahngetriebe aufweisen, wobei das Zahngetriebe eingerichtet ist, um in einem ersten Winkelstellungsbereich des Energiewandlers den mechanischen Energiespeicher aufzuladen und in einem aufgeladenen Zustand zu halten, wobei das Zahngetriebe weiterhin eingerichtet ist, um in einem zweiten Winkelstellungsbereich den mechanischen Energiespeicher freizugeben, so dass Energie für die Stechbewegung der Lanzette bereitgestellt wird. Zu diesem Zweck kann das Zahngetriebe beispielsweise ein oder mehrere teilbezahnte Zahnräder aufweisen, wobei das teilbezahnte Zahnrad in mindestens einem ersten Umfangsbereich bezahnt ist und in mindestens einem zweiten Umfangsbereich unbezahnt ist. Der Begriff des "Zahnrades" ist hierbei jedoch weit zu fassen und umfasst eine Vielzahl möglicher Zahnradsysteme, wie beispielsweise Stimradzahnräder, Kegelradzahnräder, Schneckenzahnräder, Zahnstangen, Spindelräder, Kronräder, Planetenräder oder ähnliches. Zusätzlich kann das Zahngetriebe weiterhin noch eine Sperre aufweisen, welche eingerichtet ist, um ein unerwünschtes Entladen des Energiespeichers in dem ersten Winkelstellungsbereich zu verhindern. Beispielsweise kann diese Sperre eine Sperrklinke umfassen, welche das Entladen des Energiespeichers in dem ersten Winkelstellungsbereich verhindert. Auch andere Arten von Sperren sind jedoch realisierbar.

Insgesamt kann die gesamte Antriebseinheit ein Zahnradsystem umfassen, wobei wiederum die oben genannten Zahnradsysteme einzeln oder in Kombination zum Einsatz kommen können. So kann das Zahnradsystem wiederum beispielsweise eines oder mehrere der folgenden Getriebeelemente umfassen ein Stirnradgetriebe, ein Kegelradgetriebe, ein Schneckengetriebe, ein Zahnstangengetriebe, ein Spindelradgetriebe, ein Kronradgetriebe, ein Planetengetriebe. Dabei kann das Zahnradsystem in einer Ebene angeordnet werden, wobei jedoch einzelne Elemente oder mehrere Elemente des Zahnradgetriebes auch aus dieser Ebene herausragen können, beispielsweise im Rahmen eines Kegelradgetriebes. Vorzugsweise ist dann die Ebene des Zahnradgetriebes auch die Ebene, in welcher die Bewegung des analytischen Hilfsmittels in die ausgelenkte Position, beispielsweise die Probennahmebewegung oder Probenaufnahmebewegung des Testelements und/oder die Lanzettenbewegung der Lanzette, erfolgt. Dies ermöglicht eine besonders flache Ausgestaltung des gesamten Probengewinnungssystems. Auch andere Ausgestaltungen sind jedoch denkbar, beispielsweise eine Lanzettenbewegung und/oder eine Probennahmebewegung senkrecht zu einer Ebene des Zahnradgetriebes.

In einer besonders bevorzugten Ausgestaltung der Erfindung umfasst die Kopplungsvorrichtung ein erstes Antriebsrad und ein zweites Antriebselement. Wiederum kann es sich bei diesem ersten Antriebsrad beispielsweise um eines der oben beschriebenen Zahnräder und/oder eine Kombination derartiger Zahnräder handeln. Auch das zweite Antriebselement ist vorzugsweise als Rad ausgestaltet, wobei jedoch hier auch eine Antriebsstange mit umfasst sein kann. Wiederum können eines oder mehrere der oben beschriebenen Zahngetriebe zum Einsatz kommen.

Das erste Antriebsrad ist mit dem Energiewandler gekoppelt. Diese Kopplung kann direkt oder indirekt, das heißt über ein Zwischengetriebe oder eine Zwischenkopplung, erfolgen, dergestalt, dass das erste Antriebsrad durch den Energiewandler antreibbar ist, und zwar vorzugsweise in beide Drehrichtungen. Das zweite Antriebselement ist mit der ersten Systemfunktion und/oder der zweiten Systemfunktion gekoppelt. Das erste Antriebsrad und das zweite Antriebselement sind miteinander durch das drehrichtungssensitive Element verbunden. Dieses drehrichtungssensitive Element ist dabei derart ausgestaltet, dass dieses in einer ersten Drehrichtung (zum Beispiel im Uhrzeigersinn oder im Gegenuhrzeigersinn) das erste Antriebsrad und das zweite Antriebselement miteinander koppelt und in einer zweiten Drehrichtung (zum Beispiel entgegen dem Uhrzeigersinn bzw. im Uhrzeigersinn) das erste Antriebsrad und das zweite Antriebselement voneinander entkoppelt. Durch diese Weiterbildung der Erfindung kann, durch Verwendung des ersten Antriebsrades, des zweiten Antriebselements und des drehrichtungssensitiven Elements, durch einfaches Einstellen einer bestimmten Drehrichtung eine Systemfunktion an- oder abgekoppelt werden.

Besonders bevorzugt ist es dabei, wenn zwei oder sogar mehr mit dem ersten Antriebsrad durch zwei bzw. mehrere drehrichtungssensitive Elemente verbundene zweite Antriebselemente vorgesehen sind. Dabei sollen die jeweiligen drehrichtungssensitiven Elemente derart ausgestaltet sein, dass das erste Antriebsrad in unterschiedlichen Drehrichtungen jeweils an unterschiedliche Antriebselemente ankoppelt. So kann beispielsweise das erste Antriebsrad in einer ersten Drehrichtung an ein erstes der zweiten Antriebselemente ankoppeln und in einer zweiten Drehrichtung an ein zweites der zweiten Antriebselemente. Auf diese Weise können jeweils unterschiedliche Systemfunktionen an das erste Antriebsrad angekoppelt werden, welche je nach Drehrichtung aktiviert werden können. Unter der "Drehrichtungssensitivität" des drehrichtungssensitiven Elementes ist dabei die Richtung zu verstehen, in welcher die Kopplung zwischen erstem Antriebsrad und zweitem Antriebselement erfolgt, bzw. die Richtung, in welcher eine Entkopplung erfolgt. Diese Richtungen sollen für die zwei oder mehr zweiten Antriebselemente jeweils unterschiedlich sein.

Eine weitere bevorzugte Weiterbildung der Erfindung betrifft eine bevorzugte Ausgestaltung des drehrichtungssensitiven Elements. So ist es besonders bevorzugt, wenn dieses drehrichtungssensitive Element mindestens einen Freilauf aufweist. Unter einem Freilauf ist dabei eine Drehkopplung zwischen zwei Elementen zu verstehen, welche in einer Drehrichtung eines ersten dieser Elemente eine kraft- oder formschlüssige Kopplung mit dem zweiten Element (und dadurch eine Mitnahme) bewirkt, wobei in der anderen Drehrichtung dieser Kraftschluss bzw. Formschluss aufgehoben wird. Derartige Freiläufe werden auch als Überholkupplungen bezeichnet.

Freiläufe sind in den verschiedensten Ausführungsformen aus dem Stand der Technik bekannt. Dabei ist es im Rahmen der vorliegenden Erfindung besonders bevorzugt, wenn der Freilauf einen Klemmrollenfreilauf (wobei unter "Rollen" hier sowohl Rollen als auch Kugeln zu verstehen sein können), einen Klemmkörperfreilauf, einen Sperrklinkenfreilauf, einen Reibrichtsperrenfreilauf, einen Schlepphebelfreilauf oder eine Kombination dieser und/oder anderer Arten von Freiläufen umfasst. Um das bei üblichen Freiläufen bekannte Problem des Freilauf-Totgangs zu vermindern, das heißt des Drehbereichs in Mitnahmerichtung, in welchem auch in dieser Mitnahmerichtung noch keine Mitnahme erfolgt, kann der Freilauf weiterhin mindestens eine Freilaufsperre umfassen. Derartige Freilaufsperren, welche den Freilauf-Totgang vermindern, sind aus dem Stand der Technik bekannt.

Alternativ oder zusätzlich zur beschriebenen Verwendung eines Freilaufs kann das drehrichtungssensitive Element jedoch auch auf andere Arten ausgestaltet sein bzw. andere Arten drehrichtungssensitiver Elemente umfassen. Derartige andere Ausgestaltungen drehrichtungssensitiver Elemente umfassen beispielsweise Kopplungen auf unterschiedlichen Ebenen, wobei für jeweils eine Drehrichtung eine Ebene genutzt wird. So kann beispielsweise das drehrichtungssensitive Element mindestens ein mit dem Energiewandler gekoppeltes erstes Antriebsrad und mindestens zwei mit unterschiedlichen Systemfunktionen gekoppelte, in unterschiedlichen Ebenen angeordnete zweite Antriebselemente aufweisen. Bezüglich des Ausdrucks "Antriebselemente" sei wieder auf die oben beschriebenen Möglichkeiten verwiesen. Dabei ist das erste Antriebsrad derart eingerichtet, dass dieses in der ersten Drehrichtung auf einer ersten Ebene angeordnet ist und an ein erstes der zweiten Antriebselemente gekoppelt ist, und dass das erste Antriebsrad in der zweiten Drehrichtung auf einer zweiten Ebene angeordnet ist und an ein zweites der zweiten Antriebselemente gekoppelt ist. Diese Nutzung verschiedener Ebenen kann beispielsweise dadurch erfolgen, dass das erste Antriebsrad auf einer Achse angeordnet ist, in welcher eine Nut oder ein Gewinde vorgesehen ist, in welche bzw. welches ein Eingriffselement (zum Beispiel ein Gewindeabschnitt, ein Verzahnungsabschnitt, ein Stift, ein Bolzen oder ein Dorn) des ersten Antriebsrades eingreift. In einer ersten Drehrichtung wird dann das erste Antriebsrad auf die erste Ebene "geschraubt", wohingegen in der zweiten Drehrichtung das Antriebsrad auf die zweite Ebene verbracht (geschraubt) wird. Auch andere Arten der Verbringung in unterschiedliche Ebenen sind denkbar. Auch diese vorgeschlagene Möglichkeit stellt eine Realisierung der erfindungsgemäßen Idee dar, dass unterschiedliche Drehrichtungen des Energiewandlers für die Kopplung an unterschiedliche Systemfunktionen genutzt werden können.

Auch in ein und derselben Drehrichtung können unterschiedliche Systemfunktionen miteinander gekoppelt werden. So kann beispielsweise das Probengewinnungssystem derart ausgestaltet sein, dass in der ersten Drehrichtung zwei oder mehr Systemfunktionen an den Energiewandler angekoppelt sind, oder, alternativ oder zusätzlich, auch in der zweiten Drehrichtung mindestens zwei unterschiedlich Systemfunktionen an den Energiewandler angekoppelt sind.

Besonders bevorzugt ist es im Rahmen dieser Weiterentwicklung der Erfindung, wenn in einer der Drehrichtungen (das heißt in der ersten oder der zweiten Drehrichtung) gleichzeitig eine Kopplung der Systemfunktion, in welcher ein mechanischer Energiespeicher mit Energie aufgeladen wird, und einer Systemfunktion, in welcher ein Testelement in eine ausgelenkte Position überführt wird, in welcher eine flüssige Probe auf einem Testfeld des Testelements appliziert werden kann, an den Energiewandler gekoppelt werden. Diese Kopplung von Aufladung des Energiespeichers und einer Probennahmebewegung ist insbesondere deswegen bevorzugt, weil beide Systemfunktionen in der Regel eine langsame Bewegung erfordern. So erfolgt beispielsweise eine Probennahmebewegung mittels eines Testelements, das heißt zum Beispiel ein Abholen eines Blutstropfens mittels eines auf einem Band angeordneten Testfeldes, eine Bewegung, welche üblicherweise mit einer Geschwindigkeit von einigen cm/s durchgeführt wird.

Wird eine Drehrichtung kombiniert für ein Aufladen des Energiespeichers und eine Probennahmebewegung genutzt, so ist es weiterhin besonders bevorzugt, wenn die Kopplungsvorrichtung derart eingerichtet ist, dass der Energiewandler in der zweiten Drehrichtung, welche von der ersten Drehrichtung verschieden ist, an eine Systemfunktion angekoppelt werden kann, in welcher ein analytisches Hilfsmittel in einer Applikationsposition bereitgestellt wird.

Das oben beschriebene Probengewinnungssystem in einer der möglichen Ausgestaltungen kann vorzugsweise, wie oben bereits beschrieben, im Rahmen verschiedener, sequentiell durchgeführter Betriebsphasen eingesetzt werden, welche insgesamt einen Probengewinnungszyklus bilden. Besonders bevorzugt sind dabei ein Probengewinnungssystem und ein Verfahren, welche sowohl das Erzeugen einer flüssigen Probe (zum Beispiel eine Lanzettenbewegung) und ein Abholen der flüssigen Probe mittels eines Testelements umfassen. Zu diesem Zweck wird ein Verfahren zur Gewinnung einer flüssigen Probe vorgeschlagen, welches beispielsweise auf einem Probengewinnungssystem gemäß einer der obigen Ausgestaltungen durchgeführt werden kann, sowie ein Probengewinnungssystem, welches eingerichtet ist, um das vorgeschlagene Verfahren bzw. die Probennahmesequenz durchzuführen. Die im Folgenden beschriebenen Verfahrensschritte können dabei vorzugsweise in der im Folgenden dargestellten Reihenfolge durchgeführt werden, wobei jedoch auch andere, nicht dargestellte Verfahrensschritte zusätzlich durchgeführt werden können. Alternativ sind auch andere als die dargestellte Reihenfolge möglich. Zudem kann die dargestellte Reihenfolge der Verfahrensschritte a) bis d) zyklisch oder antizyklisch permutiert werden, so dass statt der Reihenfolge a), b), c), d) auch beispielsweise die Reihenfolgen d), a), b), c); c), d), a), b) oder b), c), d), a) durchführbar sind. Weiterhin können auch einzelne oder mehrere Verfahrensschritte wiederholt durchgeführt werden, oder es können einzelne Verfahrensschritte zeitlich parallel oder zeitlich überlappend durchgeführt werden.

Das Verfahren umfasst folgende Schritte:
a) Der Energiewandler führt eine erste Drehbewegung in der ersten Drehrichtung aus, wobei ein mechanischer Energiespeicher freigegeben wird und Energie für eine Stechbewegung einer in einer Applikationsposition angeordneten Lanzette des analytischen Hilfsmittels abgibt.
b) Der Energiewandler führt eine zweite Drehbewegung in der zweiten Drehrichtung aus, wobei ein Testelement mit einem Testfeld zur Analyse der flüssigen Probe in die Applikationsposition überführt wird.
c) Der Energiewandler führt eine dritte Drehbewegung in der ersten Drehrichtung aus, wobei ein Aufladen des mechanischen Energiespeichers mit Energie erfolgt und wobei das Testelement in eine ausgelenkte Position überführt wird, um eine flüssige Probe auf dem Testfeld des Testelements zu applizieren.
d) Der Energiewandler führt eine vierte Drehbewegung in der zweiten Drehrichtung aus, wobei eine Lanzette in die Applikationsposition überführt wird.

Wie oben beschrieben, können weitere Verfahrensschritte vorgesehen sein. So kann beispielsweise zwischen den Verfahrensschritten c) und d) und/oder zu anderen Zeitpunkten des Verfahrens ein Messschritt vorgesehen sein, in welchem das Testelement, auf welches die flüssige Probe appliziert wurde, ausgewertet, d.h. gemessen, wird. Dies kann beispielsweise unmittelbar in der Applikationsposition erfolgen, wobei in diesem Fall in der Applikationsposition eine entsprechende Messeinrichtung (beispielsweise eine optische und/oder eine elektrochemische Messeinrichtung) vorgesehen wäre. Alternativ oder zusätzlich kann die Messung jedoch auch in einer anderen Position, insbesondere einer speziellen Messposition, erfolgen. Zu diesem Zweck kann beispielsweise ein separater Transportschritt vorgesehen sein, in welchem das Testelement mit der darauf applizierten Probe in die Messposition überführt wird. Dieser Transportschritt kann auch ganz oder teilweise mit anderen Verfahrensschritten zusammenfallen, beispielsweise mit dem Verfahrensschritt d).

Das beschriebene Verfahren in einer der dargestellten Varianten ermöglicht es, auf hoch integrierte Weise und vorzugsweise mittels eines einzelnen Energiewandlers, verschiedene Funktionen, welche für eine Probennahme erforderlich sind, nacheinander durchzuführen bzw. zu aktivieren. Weitere, nicht aufgeführten Verfahrensschritte, können beispielsweise die Analyse der flüssigen Probe auf dem Testfeld umfassen, beispielsweise, wie oben beschrieben, einen quantitativen Nachweis mindestens eines Analyten in der Probe mittels einer Testchemie, mittels eines optischen und/oder elektrochemischen Verfahrens. Dieser Nachweis kann ebenfalls in der Applikationsposition unmittelbar erfolgen, beispielsweise indem das Testelement in diese Applikation mit Licht bestrahlt und beispielsweise eine Farbänderung detektiert wird, oder es kann eine separate Analysenposition vorgesehen sein, in welcher das Testelement ausgewertet wird.

Neben dem Probengewinnungssystem und dem beschriebenen Verfahren in einer der dargestellten Ausführungsformen wird weiterhin eine Bandkassette vorgeschlagen, welche zum Einsatz in einem Probengewinnungssystem gemäß einem der oben beschriebenen Ausführungsbeispiele geeignet ist. Die Bandkassette umfasst ein Analyseband gemäß der obigen Beschreibung, also insbesondere ein Band, auf welchem mindestens ein Testelement und/oder mindestens eine Lanzette angeordnet sind. Weiterhin umfasst die Bandkassette mindestens einen Gutwickel, von welchem das Analyseband abgewickelt und bereitgestellt werden kann, sowie mindestens einen Schlechtwickel, also eine Rolle, auf welcher verbrauchtes Analyseband aufgewickelt werden kann. Erfindungsgemäß wird weiterhin vorgeschlagen, einen Teil des Kopplungselements in der Bandkassette zu integrieren und somit austauschbar zu gestalten. Auf diese Weise wird beim Austausch der Bandkassette die Ankopplung an das Analyseband erleichtert, da nunmehr beim Auswechseln der Bandkassette lediglich der in der Bandkassette verbleibende Teil des Kopplungselements an das restliche, nicht ausgetauschte Kopplungselement angekoppelt werden muss, nicht hingegen eine neue Ankopplung an das empfindliche Band erforderlich ist. Insbesondere kann beispielsweise das (vorzugsweise mit dem Analyseband unmittelbar wechselwirkende) Koppelstück als auswechselbares Teil der Bandkassette ausgestaltet sein. Daneben kann die Bandkassette weitere Bestandteile umfassen, beispielsweise ein Gehäuse, eine Grundplatte und weitere Elemente. Die Bandkassette kann insbesondere von ihren mechanischen Dimensionen zum Einlegen oder Einschieben in das Probengewinnungssystem, beispielsweise in ein Gehäuse des Probengewinnungssystems, geeignet sein und kann als Einwegmaterial oder auch als mehrfach verwendbare, d.h. rezyklierbare, Bandkassette ausgestaltet sein.

### Ausführungsbeispiele

Weitere Einzelheiten und Merkmale der Erfindung ergeben sich aus der nachfolgenden Beschreibung von bevorzugten Ausführungsbeispielen in Verbindung mit Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigt:
- Figuren 1A und 1B: ein Grundkonzept eines Probengewinnungssystems mit einem Analyseband;
- Figur 1C: ein Ausführungsbeispiel eines Analysebandes;
- Figuren 2A bis 2C: verschiedene perspektivische Darstellungen eines Ausführungsbeispiels eines Probengewinnungssystems;
- Figuren 3A bis 3D: einen Probengewinnungszyklus zur Verdeutlichung eines Ausführungsbeispiels eines mit dem Probengewinnungssystem gemäß den Figuren 2A bis 2C durchgeführten Verfahrens zum Gewinnen einer flüssigen Probe;
- Figur 4: ein alternatives Ausführungsbeispiel eines drehrichtungssensitiven Elements; und
- Figuren 5A bis 9: mehrere Ausführungsbeispiele von Freiläufen, die als drehrichtungssensitive Elemente eingesetzt werden können.

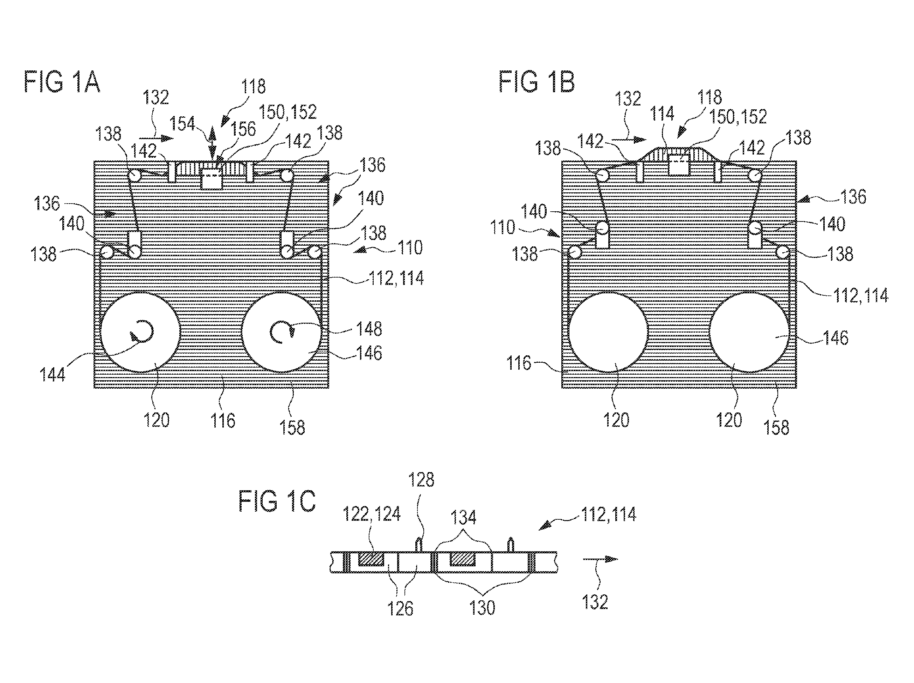

In den Figuren 1A und 1B ist ein Grundkonzept eines Ausführungsbeispiels eines erfmdungsgemäßen Probengewinnungssystems 110 schematisch in Draufsicht dargestellt. Das Probengewinnungssystem verwendet dabei ein analytisches Hilfsmittel 112 in Form eines Analysebandes. Ein Ausführungsbeispiel eines derartigen Analysebandes 114 ist exemplarisch in Figur 1C dargestellt.

Das Probengewinnungssystem ist in diesen Figuren 1A und 1B, sowie auch in den nachfolgenden Figuren, lediglich exemplarisch und ausschnittsweise dargestellt und kann weitere, nicht dargestellte Komponenten umfassen. Insbesondere können weitere elektronische Komponenten umfasst sein, beispielsweise Komponenten zur Durchführung und Auswertung einer qualitativen und/oder quantitativen Analyse einer Probe (zum Beispiel eine elektronische Steuerung, Anzeigenelemente, Ein- und Ausgabemittel und/oder weitere Komponenten). Die in den Figuren dargestellten Bestandteile des Probengewinnungssystems 110 sind auf einer Grundplatte 116 montiert. Daneben können weitere Komponenten vorhanden sein, insbesondere ein die Grundplatte 116 umschließendes Gehäuse, beispielsweise ein Gehäuse, welches in einer Applikationsposition 118 des Probengewinnungssystems 110 eine Öffnung (zum Beispiel eine durch eine Klappe und/oder einen Schieber verschließbare Öffnung) aufweist, durch welche ein Zugriff auf das analytische Hilfsmittel 112 hergestellt werden kann.

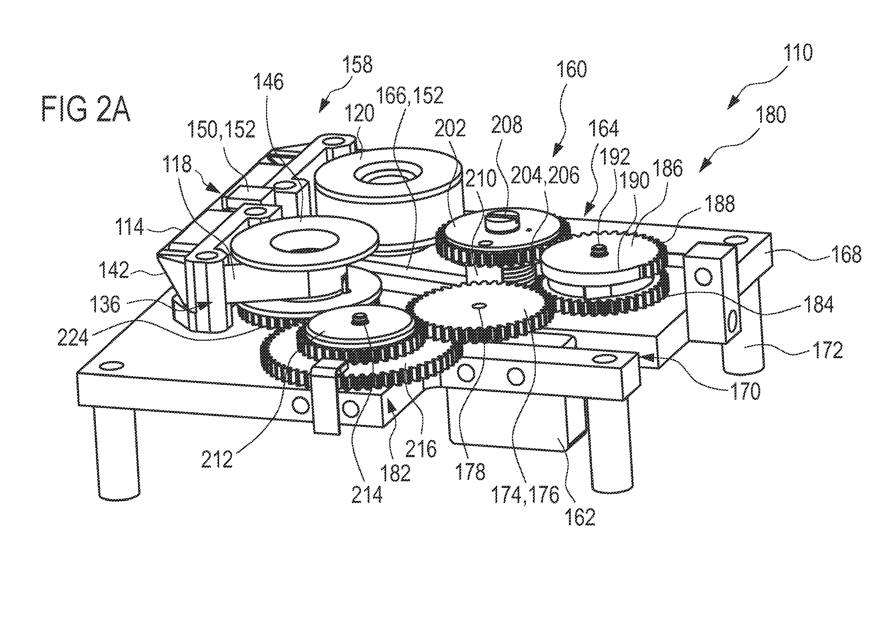

In den Figuren 1A und 1B ist lediglich eine schematische Führung des Analysebandes 114 dargestellt. Nicht dargestellt sind weitere Einzelheiten eines Antriebsystems, welche aus den nachfolgenden Figuren 2A bis 3D näher hervorgehen.

Wie in den Figuren 1A und 1B gezeigt, ist das Analyseband 114 zunächst auf einem Gutwickel 120 aufgenommen, welcher als Spule mit aufgewickeltem Analyseband 114 ausgestaltet ist. Wie aus der Darstellung eines Abschnitts dieses Analysebands 114 in Figur 1C hervorgeht, umfasst das Analyseband 114 alternierend angeordnete Testelemente 122 in Form von Testfeldern 124. Diese Testfelder 124 können beispielsweise auf ein Trägerband 126 aufgedruckt und/oder auflaminiert und/oder aufgeklebt sein und enthalten eine Testchemie, welche bei Kontakt mit dem mindestens einen nachzuweisenden Analyten mindestens eine chemische oder physikalische Eigenschaft ändert. Beispielsweise kann es sich dabei um eine elektrochemisch nachweisbare Eigenschaft und/oder eine optisch nachweisbare Eigenschaft handeln, beispielsweise eine Farbänderung.

Zwischen den Testelementen 122 sind jeweils Lanzetten 128 auf das Trägerband 126 aufgebracht. Dabei sind die Testfelder 124 und die Lanzetten 128 derart auf dem Trägerband 126 angeordnet, dass diese zur selben Seite dieses Trägerbandes 126 hinweisen. Jede dieser Lanzetten 128 und Testfelder 124 bzw. Testelemente 122 bilden für sich genommen ein einzelnes analytisches Hilfsmittel 112, so dass sowohl die Gesamtheit der analytischen Hilfsmittel als auch ein einzelnes analytisches Hilfsmittel 112 im Rahmen der vorliegenden Erfindung gleich bezeichnet werden kann. Bei dem dargestellten bevorzugten Ausführungsbeispiel, bei welchem Lanzetten 128 und Testfelder 124 verwendet werden, bilden jeweils eine Lanzette 128 und ein Testelement 122, ein einander zugeordnetes Paar 130 analytischer Hilfsmittel 112, wobei in einer Laufrichtung 132 des Analysebandes 114 jeweils das der Lanzette 128 zugeordnete Testfeld 124 der Lanzette 128 nachgeordnet ist. Zwischen den einzelnen analytischen Hilfsmitteln 112, also zwischen den Lanzetten 128 und den Testfeldern 124, sind auf dem Trägerband 126 Markierungen 134 aufgedruckt. Diese Markierungen 134 können beispielsweise von dem Probengewinnungssystem 110 optisch erkannt werden, um auf diese Weise ein bestimmtes analytisches Hilfsmittel 112 des Analysebands 114 in der Applikationsposition 118 zu positionieren.

Auch andere Ausgestaltungen des analytischen Hilfsmittels 112, des Analysebands 114 und der Lanzetten 128 und Testelemente 122 als die in Figur 1C dargestellte Ausführungsform sind jedoch denkbar. So ist beispielsweise auch ein Analyseband 114 möglich, bei welchem lediglich Testfelder 124 aufgebracht sind, ohne dass Lanzetten 128 vorhanden sind, oder ein Analyseband 114, welches lediglich Lanzetten 128 umfasst.

Von dem Gutwickel 120 in den Figuren 1A und 1B wird das Analyseband 114 über ein Rollensystem 136, welches feste Rollen 138 und bewegliche Rollen 140 umfasst, zu der Applikationsposition 118 geführt. Dabei ist das Rollensystem 136 derart ausgestaltet, dass kurz vor Erreichen der Applikationsposition 118 durch ein Umlenksystem 142, welches Umlenkrollen umfasst, in seiner Ausrichtung umgelenkt wird. Während das Analyseband 114 außerhalb der Applikationsposition 118 durch das Rollensystem 136 im Wesentlichen in einer Ebene geführt wird, in welcher das Analyseband 114 senkrecht zur Zeichenebene in den Figuren 1A und 1B (das heißt senkrecht zur Grundplatte 116) angeordnet ist, wird dieses Analyseband 114 durch das Umlenksystem 142 in der Applikationsposition 118 in eine im Wesentlichen parallele Ausrichtung zur Grundplatte 116 gebracht. Diese parallele Ausrichtung gewährleistet, dass eine Auslenkung des Analysebandes 114 in der Applikationsposition 118 (siehe unten, vgl. die Figuren 1A und 1B) eine Seitenkante des Analysebandes 114 (in Figur 1C die obere Kante des Analysebandes114) hin zu einer Probennahme bewegen kann. Beispielsweise kann dies, wie unten näher ausgeführt wird, zur Aufnahme einer flüssigen Probe auf das Testfeld 124, welches in der Applikationsposition 118 angeordnet ist, genutzt werden, oder um eine Lanzettenbewegung mittels derjenigen Lanzette 128, welche in der Applikationsposition 118 angeordnet ist, durchzuführen.

Die Bewegungsrichtung des Analysebandes 114 in den Figuren 1A und 1B ist mit dem Pfeil 132 gekennzeichnet. Nacheinander werden also die einzelnen analytischen Hilfsmittel 112 in die Applikationsposition 118 verbracht (getaktet), wobei der Gutwickel 120 in einer Abwickelrichtung 144 (in Figur 1A im Uhrzeigersinn) gedreht wird. Verbrauchte analytische Hilfsmittel 112, das heißt Testfelder 124, auf welche eine flüssige Probe appliziert wurde bzw. Lanzetten 128, welche für eine Lanzettenbewegung genutzt wurden, werden anschließend über das Rollensystem 136 auf einen Schlechtwickel 146, welcher in einer Aufwickelrichtung 148 gedreht wird (in diesem Ausführungsbeispiel ebenfalls im Uhrzeigersinn) verbracht. Auf diese Weise ist beispielsweise sichergestellt, dass diese analytischen Hilfsmittel 112 nicht wieder verwendet werden, sondern sicher und hygienisch und ohne Gefahr für einen Benutzer entsorgt sind.

Weiterhin ist in den Figuren 1A und 1B ein Koppelstück 150 dargestellt, welches Teil eines Kopplungselements 152 (in den Figuren 1A und 1B nicht vollständig dargestellt, siehe die nachfolgenden Figuren) bildet und welches in einer Auslenkungsrichtung 154 (in Figur 1A durch einen Doppelpfeil dargestellt) beweglich gelagert ist. Die Auslenkungsrichtung 154 ist dabei in der Applikationsposition 118 im Wesentlichen senkrecht zur Bewegungsrichtung 132 des Analysebandes 114. Die Figur 1A zeigt dabei eine nicht ausgelenkte Position des Kopplungsstücks 150 und des analytischen Hilfsmittels 112 in der Applikationsposition 118, wohingegen die Figur 1B eine ausgelenkte Position dieser Elemente zeigt. Das Koppelstück 150 kann beispielsweise einen Schlitz 156 umfassen, welcher in das Koppelstück 150 eingelassen ist und durch welchen das Analyseband 114 derart geführt ist, dass die Applikationskante (das heißt die obere Kante in den Figuren 1A und 1B) zugänglich bleibt, beispielsweise für eine Aufbringung einer flüssigen Probe und/oder einer Lanzettenbewegung. Durch diesen Schlitz 156 kann das Analyseband 114 gleitend geführt werden.

Das Koppelstück 150 ist dabei an weitere Teile des Kopplungselements 152 angeschlossen, welche eine Auslenkung des Analysebandes 114 in der Applikationsposition 118 ermöglichen und welche durch eine Antriebseinheit (siehe die nachfolgenden Figuren) angetrieben werden, um die Auslenkungsbewegung zu bewerkstelligen. Die beweglichen Rollen 140 dienen dabei dem Zweck, eine Spannungsbildung innerhalb des Analysebandes 114 bei der Auslenkung (siehe Figur 1B) zu verhindern, da sich diese beweglichen Rollen 140 in der ausgelenkten Position in einem oberen Zustand befinden (vgl. Figur 1B gegenüber Figur 1A), wodurch die durch die Auslenkung erzeugte Spannung auf dem Analyseband 114 ausgeglichen wird.

Der gesamte in den Figuren 1A und 1B gezeigte Teil des Probengewinnungssystems 110 kann beispielsweise durch separates Einbringen einzelner oder mehrerer dieser Elemente in das Probengewinnungssystem 110 erzeugt werden. So wäre es beispielsweise denkbar, den Gutwickel 120 und den Schlechtwickel 146 separat in das Probengewinnungssystem 110 einzubringen und das Analyseband 114 entsprechend durch das Rollensystem 136 zu fädeln, ähnliche zum Einfädeln eines Films in eine Kamera. Auf diese Weise könnte ein verbrauchtes Analyseband 114 gegen ein unverbrauchtes, frisches Analyseband 114 ausgetauscht werden.

Da dieses Einfädeln jedoch insbesondere für Patienten mit körperlichen Behinderungen, wie sie bei Diabetes in vielen Fällen anzutreffen sind, nur schwer zu handhaben wären, ist es bevorzugt, den in Figur 1A und 1B dargstellten Teil des Probengewinnungssystem 110 als Bandkassette 158 auszugestalten. In diesem Fall umfasst vorzugsweise die Bandkassette 158 zumindest die optionale Grundplatte 116, den Gutwickel 120, den Schlechtwickel 146 und zumindest Teile des optionalen Rollensystems 136. Auch das Koppelstück 150 kann entsprechend Teil dieser Bandkassette 158 sein. Weiterhin kann (in den Figuren 1A und 1B nicht dargestellt) die Bandkassette 158 ein Gehäuse umfassen, welches vorzugsweise ein Ankoppeln weiterer Elemente des Probengewinnungssystems 110 an den Gutwickel 120, den Schlechtwickel 146 und gegebenenfalls das Koppelstück 150 ermöglicht. Auf diese Weise kann die Bandkassette 158 vollständig als separates Bauteil in das Probengewinnungssystem 110 eingebracht werden und dort beispielsweise an eine Antriebseinheit und/oder weitere Teile des Kopplungselements 152 angekoppelt zu werden. Auf diese Weise lassen sich auch durch körperbehinderte Patienten leicht frische analytische Hilfsmittel 112 in das Probengewinnungssystem 110 einbringen, indem eine vollständige Bandkassette 158 ausgetauscht wird.

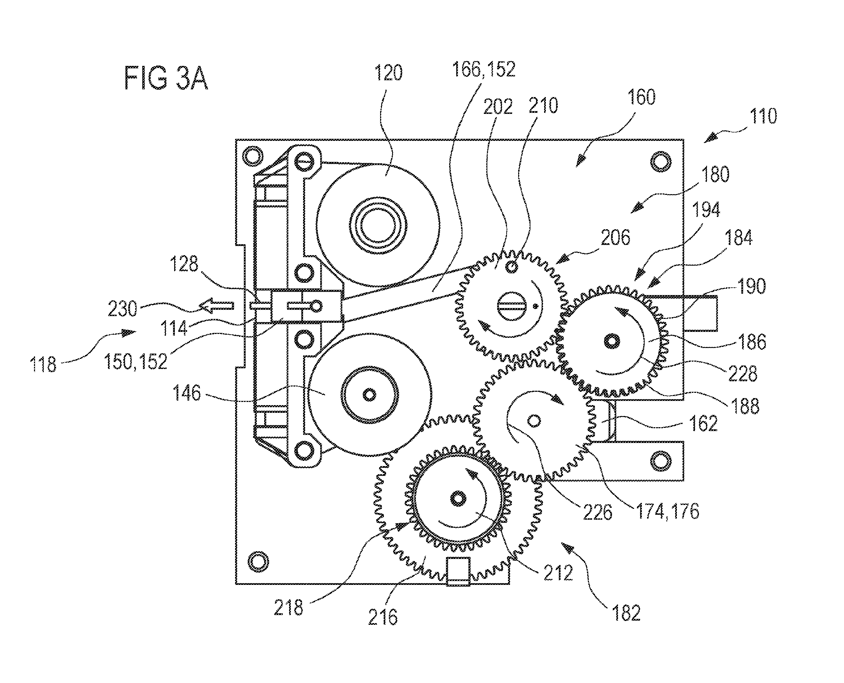

In den Figuren 2A bis 2C sind verschiedene perspektivische Darstellungen eines Ausfiihrungsbeispiels eines erfindungsgemäßen Probengewinnungssystems 110 dargestellt, dessen Funktionsweise anschließend anhand einer in den Figuren 3A bis 3D dargestellten Sequenz erläutert wird. Das Probengewinnungssystem 110 ist vorzugsweise eingerichtet für den Betrieb mit einer Bandkassette 158 für ein Analyseband 114, welche beispielsweise analog zu dem Beispiel in den Figuren 1A und 1B ausgestaltet sein kann. Diese Bandkassette 158 ist in den Figuren 2A bis 2C jedoch lediglich teilweise dargestellt, so dass beispielsweise die Grundplatte 116 nicht gezeigt ist, da diese weitere wesentliche Elemente der Bandkassette 158 verdecken würde. Diese Grundplatte würde sich in den Figuren 2A bis 2C oberhalb des dargestellten Aufbaus befinden.

Wiederum umfasst die Bandkassette 158 einen Gutwickel 120, einen Schlechtwickel 146 sowie ein Rollensystem 136, ein Umlenksystem 142 und ein Koppelstück 150. Dabei ist das Rollensystem 136 in den Figuren 2A bis 2C lediglich ansatzweise dargestellt und leicht abweichend von dem in den Figuren 1A und 1B dargestellten Rollensystem 136 aufgebaut. Es ist jedoch analog auch möglich, ein Rollensystem 136 gemäß den Figuren 1A und 1B zu verwenden, insbesondere ein Rollensystem 136 mit festen Rollen 138 und beweglichen Rollen 140, welche einen Spannungsaufbau innerhalb des Analysebandes 114 verhindern. Ebenfalls nicht dargestellt in den Figuren 2A bis 2C ist ein mögliches Gehäuse der Bandkassette 158, sowie wiederum weitere Bestandteile des Probengewinnungssystems 110, wie beispielsweise eine elektronische Steuerung, sowie Eingabemittel, Anzeigenelemente, Datenspeicher, ein Gehäuse des Probengewinnungssystems 110, Auswertungsvorrichtungen zur qualitativen und/oder quantitativen Analyse der flüssigen Probe, ein Gehäuse des Probengewinnungssystem 110 oder ähnliche Elemente.

Das Probengewinnungssystem 110 in den Figuren 2A bis 2C umfasst weiterhin eine Antriebseinheit 160 zum Antreiben der Funktionen des Probengewinnungssystems 110. Die Antriebseinheit 160 wiederum umfasst einen Energiewandler, welcher hier ohne Beschränkung der Möglichkeiten der Ausgestaltung des Energiewandlers vorzugsweise als elektromechanischer Energiewandler in Form eines Motors 162 ausgestaltet ist. Der Motor 162 ist vorzugsweise als Elektromotor ausgestaltet. Weiterhin umfasst die Antriebseinheit 160 ein Zahngetriebe 164, welches an ein Kopplungselement 152 ankoppelt. Dieses Kopplungselement 152 umfasst neben dem bereits in den Figuren 1A und 1B aufgeführten Koppelstück 150, welches Bestandteil der Bandkassette 158 bildet, als weiteres Element in dem in den Figuren 2A bis 2C dargestellten Ausführungsbeispiel als Verbindungselement eine Pleuelstange 166, welche an das Koppelstück 150 ankoppelt und die Auslenkungsbewegung entlang der Auslenkungsrichtung 154 in den Figuren 1A und 1B bewirken kann.

Die gesamte Antriebseinheit 160 ist in dem in den Figuren 2A bis 2C dargestellten Ausführungsbeispiel auf einer Basisplatte 168 aufgebracht. Dabei ist der Motor 162 in einer Motorführung 170 in Form eines Schlitzes in der Basisplatte 168 fixiert, derart, dass ein großer Teil des Körpers dieses Motors 162 unterhalb der Basisplatte 168 (das heißt auf der dem Zahnradgetriebe 164 gegenüberliegenden Seite der Basisplatte 168) angeordnet ist. Weiterhin verfügt die Basisplatte 168 über Stützen 172, über welche die Basisplatte 168 beispielsweise in einem Gehäuse des Probengewinnungssystems 110 montiert werden kann. Neben dieser in den Figuren 2A bis 2C beispielhaft dargestellten Ausführungsform der Montage der Antriebseinheit 160 sind jedoch zahlreiche weitere Möglichkeiten denkbar, diese Antriebseinheit aufzubauen bzw. zu fixieren, so dass das Beispiel lediglich Illustrationszwecken dienen soll.

Das Zahnradgetriebe 164 umfasst eine Vielzahl von auf verschiedene Weise miteinander gekoppelten Zahnrädern. Ausgangspunkt ist ein Motorzahnrad 174, welches als erstes Antriebsrad fungiert und welches drehfest mit einer Motorachse 178 des Motors 162 verbunden ist. Dieses Motorzahnrad 174 steht mit einem ersten Antriebsstrang 180 und einem zweiten Antriebsstrang 182 in Verbindung, welche unterschiedliche Systemfunktionen antreiben.

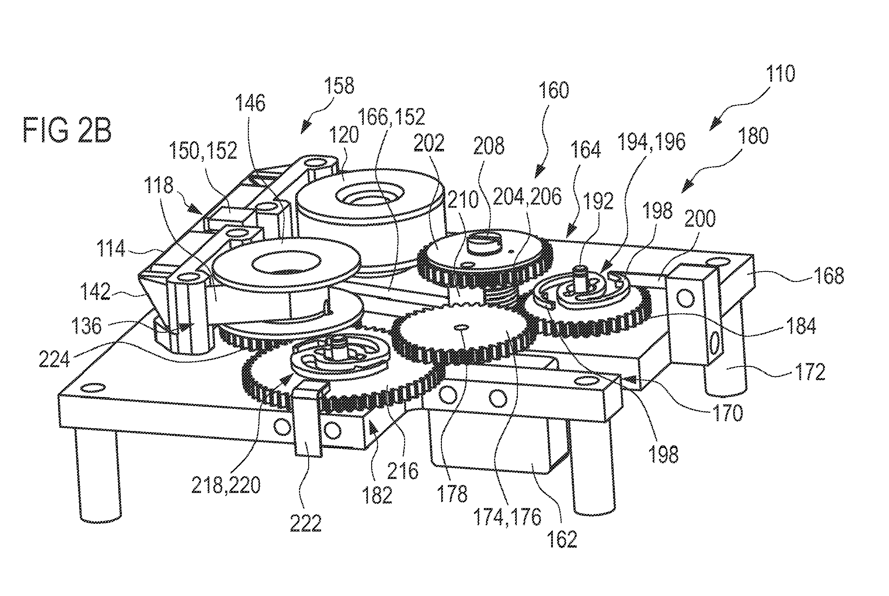

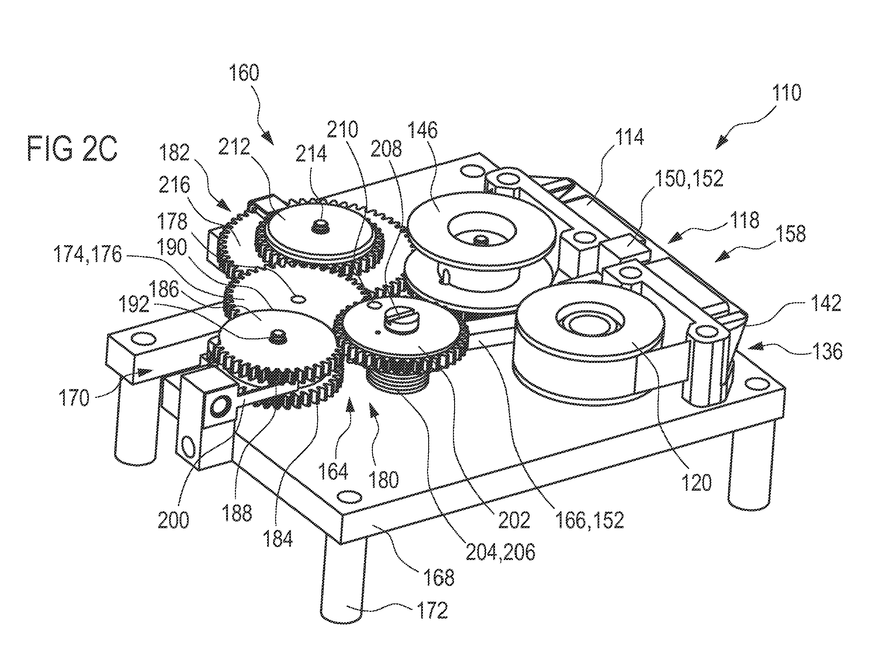

Der erste Antriebsstrang 180 umfasst ein Stechantriebszahnrad 184, welches unmittelbar in das Motorzahnrad 174 eingreift und sich somit gegensinnig zu diesem Motorzahnrad 174 dreht. Oberhalb dieses Stechantriebszahnrades 184 umfasst der erste Antriebsstrang 180 weiterhin ein Auslöserzahnrad 186 (lediglich in den Figuren 2A und 2C) dargestellt, in Figur 2B weggelassen), welches lediglich über knapp die Hälfte seines Umfanges bezahnt ist und somit einen ersten, bezahnten Umfangsbereich 188 und einen zweiten, unbezahnten Umfangsbereich 190 aufweist. Das Auslöserzahnrad 186 ist mit dem Stechantriebszahnrad zwar auf einer gemeinsamen Achse 192 gelagert, ist mit dieser Achse 192 jedoch nicht drehfest verbunden, sondern auf dieser lediglich gleitend gelagert. Weiterhin sind das Auslöserzahnrad 186 und das Stechantriebszahnrad 184 über ein zwischen diesen Elementen angeordnetes erstes drehrichtungssensitives Element 194 in Form eines ersten Freilaufs 196 verbunden. Dieser erste Freilauf 196 ist in der Darstellung gemäß Figur 2B, in welcher das Auslöserzahnrad 186 weggelassen wurde, zu erkennen und ist ebenfalls auf der Achse 192 gelagert und drehfest mit dem Stechantriebszahnrad 184 verbunden. Der Freilauf 196 verfügt über Freilaufarme, welche sich, ausgehend von der Achse 192, spiralförmig im Gegenuhrzeigersinn um diese Achse herum erstrecken. Diese Freilaufarme 198 bzw. die Enden dieser Freilaufarme 198 greifen in (in den Figuren 2A bis 2C nicht erkennbare) Mitnahmestifte oder anders gestaltete Mitnahmeelemente auf der Unterseite des Auslöserzahnrades 196 ein. Dies bedeutet, dass, wenn sich das Stechantriebszahnrad 184 in den Figuren 2A bis 2C im Gegenuhrzeigersinn dreht, dieses Stechantriebszahnrad 184 das Auslöserzahnrad 186 mitnimmt, wohingegen in entgegengesetzter Drehrichtung diese beiden Zahnräder 184, 186 voneinander entkoppelt sind. In anderen Worten treibt das Motorzahnrad 174 lediglich bei einer Bewegung im Uhrzeigersinn auch das Auslöserzahnrad 186 an, wohingegen bei einer entgegengesetzten Drehrichtung das Auslöserzahnrad 186 von dem Motorzahnrad 174 entkoppelt ist und sich nicht mitdreht.

Diese in Figur 2B dargestellte Ausgestaltung des ersten drehrichtungssensitiven Elements 194 stellt lediglich eine Möglichkeit der Ausgestaltung derartiger drehrichtungssensitiver Elemente dar, welche eine Mitnahme in einer Richtung bewirken, jedoch in einer anderen Drehrichtung eine Entkopplung. So könnten beispielsweise auch andere Arten von Freiläufen verwendet werden, beispielsweise Freiläufe mit einer höheren Anzahl an Freilaufarmen 198 oder einer anderen Ausgestaltung dieser Freilaufarme. Freiläufe mit derartigen Freilaufarmen 198, welche beispielsweise als Arme oder Zacken oder Sperrklinken ausgestaltet sein können, werden im Rahmen der vorliegenden Erfindung allgemein als Sperrklinken-Freilaufarme bezeichnet. Da ein allzu weites Spreizen der Freilaufarme 198 zu einer starken Geräuschbildung im Betrieb des Probengewinnungssystems 110 führen würde und auch ein erhöhter Totgang des ersten Freilaufs 196 auftreten würde, ist es von Vorteil, eine Freilaufsperre vorzusehen, welche den Totgang vermindert und die Geräuschbildung verringert. Eine derartige optionale Freilaufsperre ist in der dargestellten Ausführung für den ersten Freilauf 196 nicht vorgesehen, könnte jedoch zusätzlich implementiert werden.

Weiterhin umfasst der erste Antriebsstrang 180 ein Stechfederzahnrad 202, welches (zumindest in einigen Winkelstellungen) mit dem Auslöserzahnrad 186 in Eingriff steht. Dieses Stechfederzahnrad 202 ist drehfest verbunden mit einem mechanischen Energiespeicher 204 in Form einer spiralförmigen Stechfeder 206. Ein Ende dieser Stechfeder 206 ist dabei mit dem Stechfederzahnrad 202 verbunden, wohingegen ein anderes Ende dieser Stechfeder 206 ortsfest ist. Dabei ist die Stechfeder 206 um eine Achse 208 des Stechfederzahnrades 202 derart angeordnet, dass eine Drehung des Stechfederzahnrades 202 im Uhrzeigersinn ein Spannen der Stechfeder 206 und somit ein Aufladen des mechanischen Energiespeichers 204 bewirkt, wohingegen eine Drehung des Stechfederzahnrades 202 im Gegenuhrzeigersinn eine Entspannung der Stechfeder 206 und somit ein Entladen des mechanischen Energiespeichers 104 bewirkt.

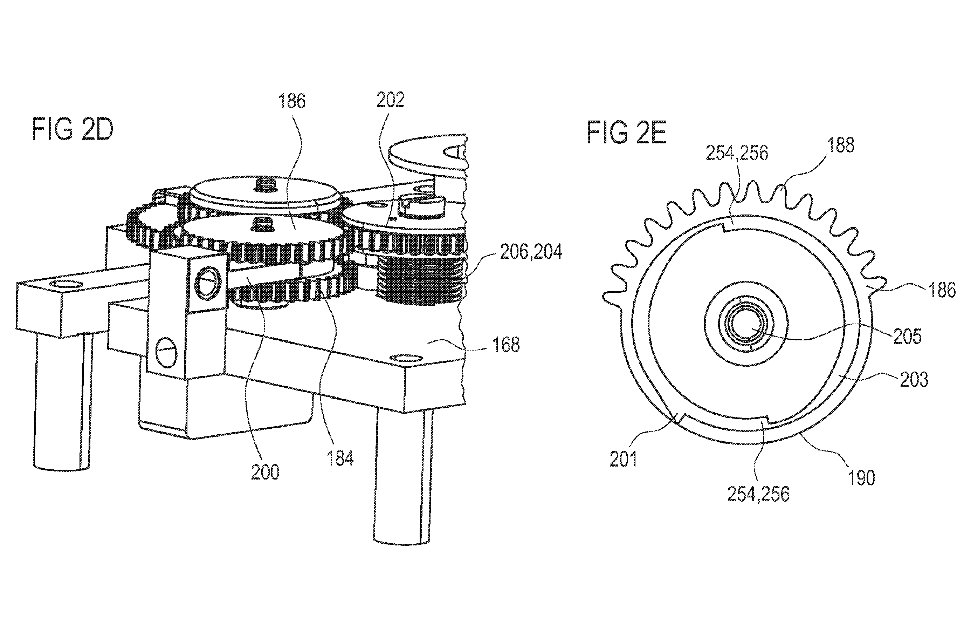

Weiterhin ist an dem Auslöserzahnrad 186 eine Sperre 200 vorgesehen, welche fest mit der Basisplatte 168 verbunden ist und deren Funktion anhand der Figuren 2D und 2E erläutert werden soll. Dabei zeigt Figur 2D eine perspektivische Teildarstellung des ersten Antriebsstrangs 180, welche verdeutlich, dass die Sperre 200 in das Auslöserzahnrad 186 eingreift. Die Sperre 200 dient dazu, in einem gespannten Zustand der Stechfeder 206 ein schlagartiges Entspannen dieser Stechfeder 206 durch eine Drehung des Stechfederzahnrades 202 (Drehung im Gegenuhrzeigersinn) und des Auslöserzahnrades 186 (Drehung im Uhrzeigersinn) zu verhindern. Die Sperre 200 ist beispielsweise elastisch (z.B. als Blattfeder) ausgestaltet und greift an der Unterseite des Auslöserzahnrades 186 an einer umfangsseitig herausragenden Sperrklinke 201 an. Diese Sperrklinke 201 ist in Figur 2E zu erkennen, welche eine Ansicht des Auslöserzahnrades 186 von unten zeigt. Die Sperrklinke 201 ist auf der Außenseite eines Klinkenringes 203 angeordnet, welcher auf der Unterseite des Auslöserzahnrades 186, also auf der den Freilaufarmen 198 zuweisenden Seite dieses Auslöserzahnrades 186, angeordnet ist. Auf seiner Innenseite weist der Klinkenring 203 eine Zahnstruktur 154 mit zwei Zähnen 256 auf, welche mit den zwei Freilaufarmen 198 zusammenwirken und mit diesen das erste drehrichtungssensitive Element 194 bilden. Weiterhin weist das Auslöserzahnrad 186 in dieser Darstellung eine Bohrung 205 zur Aufnahme der Achse 192 auf. Die Sperrklinke 201 ist umfangsseitig so positioniert, dass sie in gespannter Stellung der Stechfeder 206 (beispielsweise formschlüssig) durch die Sperre 200 gehalten wird, so dass nur eine Drehung des Auslöserzahnrades 186 im Gegenuhrzeigersinn möglich ist. Der Auslösermechanismus wird im weiteren Detail unten anhand der Figuren 3A bis 3D erläutert.

Das Stechfederzahnrad 202 ist über einen Exzenterbolzen 210 mit der Pleuelstange 166 des Kopplungselements 152 verbunden, so dass eine Drehbewegung des Stechfederzahnrades 202 unmittelbar in eine Bewegung der Pleuelstange 166 umgesetzt werden kann, und somit, über das Koppelstück 150, in eine Auslenkung des in der Applikationsposition 118 befindlichen analytischen Hilfsmittels 112. Wie oben beschrieben, können diese Auslenkungen dabei für die unterschiedlichen analytischen Hilfsmittel 112 unterschiedlich ausgestaltet sein, so dass beispielsweise für eine Lanzette 128 eine andere Auslenkung (d.h. z.B. eine andere Auslenkungsweite) erfolgen kann als für ein Testelement 122. Dies kann auf verschiedene Weisen erfolgen, beispielsweise dadurch, dass ein Kulissenantrieb mit unterschiedlichen Kulissen für die Lanzette 128 und das Testelement 122 verwendet werden. Auch beispielsweise die Positionierung der Lanzette 128 relativ zum Analyseband 114 beeinflusst die Einstechtiefe. Verschiedene andere Ausführungsvarianten sind denkbar.

Der erste Antriebsstrang 180 dient somit im Wesentlichen der Auslenkung des analytischen Hilfsmittels 112, beispielsweise für eine Stechbewegung einer Lanzette 128 und/oder eine Probennahmebewegung eines Testelements 122. Diese verschiedenen Systemfunktionen werden durch den ersten Antriebsstrang 180 an das Motorzahnrad 174 und damit an den Motor 162 angekoppelt. Wie oben beschrieben, erfolgt diese Kopplung lediglich in dem Fall, in welchem sich das Motorzahnrad 174 im Uhrzeigersinn dreht.

Weiterhin umfasst das Probengewinnungssystem 110 gemäß den Figuren 2A bis 2C noch den zweiten Antriebsstrang 182. Dieser Antriebsstrang 182 umfasst zunächst ein Wickelantriebszahnrad 212, welches in Eingriff steht mit dem Motorzahnrad 174. Dreht sich das Motorzahnrad 174 beispielsweise im Uhrzeigersinn, so dreht sich das Wickelantriebszahnrad 212 im Gegenuhrzeigersinn.

Gemeinsam mit dem Wickelantriebszahnrad 212 ist auf einer Achse 214 des Wickelantriebszahnrades 212 ein Transportzahnrad 216 aufgenommen, welches mit dem Wickelantriebszahnrad 212 jedoch nicht drehfest verbunden ist. Wiederum sind das Wickelantriebszahnrad 212 und das Transportzahnrad 216 miteinander über ein zweites drehrichtungssensitives Element 218 verbunden, welches in diesem Ausführungsbeispiel wiederum als zweiter Freilauf 220 ausgestaltet sein kann. Für die Ausgestaltung dieses zweiten Freilaufs 220 kann auf die obige Beschreibung des ersten Freilaufs 196 verwiesen werden, so dass beide Freiläufe 196, 220 beispielsweise bauteilidentisch ausgestaltet sein können. Wiederum kann auch zur Verminderung des Totgangs und zur Unterdrückung der Geräuschentwicklung eine Freilaufsperre vorgesehen sein, welche in Figur 2B lediglich angedeutet ist und welche mit der Bezugsziffer 222 bezeichnet ist.

Der zweite Freilauf 220 ist dabei derart ausgestaltet, dass dieser das Transportzahnrad 216 lediglich dann mitnimmt, wenn sich das Wickelantriebszahnrad 212 im Uhrzeigersinn dreht. Dreht sich das Wickelantriebszahnrad 212 hingegen im Gegenuhrzeigersinn, so erfolgt keine Kopplung zwischen diesem Wickelantriebszahnrad 212 und dem Transportzahnrad 216. Oder, es erfolgt, im Drehsinn des Motorzahnrades 174 ausgedrückt, lediglich eine Kopplung dieses Motorzahnrades 174 an das Transportzahnrad 216, wenn sich das Motorzahnrad 174 im Gegenuhrzeigersinn dreht. Der zweite Antriebsstrang 182 ist also lediglich bei einer Drehung des Motorzahnrades 174 im Gegenuhrzeigersinn an den Antrieb durch den Motor 162 angekoppelt, bei einer Drehung des Motors 162 im Uhrzeigersinn hingegen von diesem entkoppelt.

Das Transportzahnrad 216 steht wiederum, wie beispielsweise aus Figur 2B zu erkennen ist, in Eingriff mit einem Wickelzahnrad 224. Dieses Wickelzahnrad 224 ist drehfest (beispielsweise durch eine Verzahnung) mit dem Schlechtwickel 146 verbunden und treibt diesen an. Da bei der dargestellten Ausführungsform der Antriebseinheit 160 lediglich ein Antrieb des Transportzahnrades 216 durch den Motor 162 im Uhrzeigersinn möglich ist, ist lediglich ein Antrieb des Schlechtwickels 146 im Gegenuhrzeigersinn möglich, also eine Drehbewegung dieses Schlechtwickels 146, bei welcher das Analyseband 114 auf diesen Schlechtwickel 146 aufgewickelt wird. Es sei hierbei darauf hingewiesen, dass exemplarisch bei dem in den Figuren 2A bis 2C dargestellten Ausführungsbeispiel des Probengewinnungssystems 110 lediglich der Schlechtwickel 146 angetrieben wird. Alternativ wäre jedoch auch eine andere Ausgestaltung des Antriebes denkbar, beispielsweise eine Ausgestaltung, bei welcher lediglich der Gutwickel 120 angetrieben würde (wobei das Aufwickeln auf dem Schlechtwickel 146 beispielsweise durch ein Federantrieb dieses Schlechtwickels 146 erfolgen könnte), oder ein kombinierter Antrieb, bei welchem sowohl Gutwickel 120 als auch Schlechtwickel 146 angetrieben werden.

Die Funktionsweise des in den Figuren 2A bis 2C dargestellten Probengewinnungssystems 110 und ein erfindungsgemäßes Verfahren zum Gewinnen einer flüssigen Probe sollen anhand der Figuren 3A bis 3D im Folgenden Schritt für Schritt dargestellt werden. Diese Figuren 3A bis 3D zeigen das in den Figuren 2A bis 2C dargestellte Probengewinnungssystem 110 in Draufsicht, so dass für die Funktionsweise und Ausgestaltung der einzelnen Elemente weitgehend auf diese Figuren und die Beschreibung dieser Figuren verwiesen werden kann.

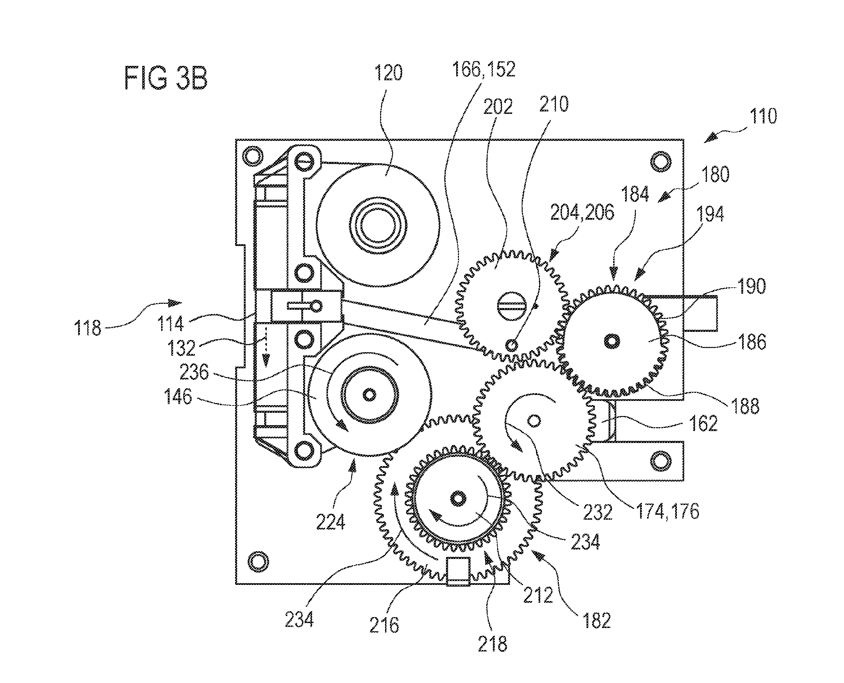

Ausgangspunkt ist dabei die in Figur 3A dargestellte Situation des Probengewinnungssystems 110. Da es sich bei dem vorgeschlagenen Verfahren jedoch vorzugsweise um ein zyklisches Verfahren handelt, bei welchem die einzelnen Verfahrensschritte (Betriebsphasen) nacheinander jeweils wiederholt durchgeführt werden, wäre es genauso gut möglich, das Verfahren auch an einem anderen Punkt, beispielsweise mit Figur 3B, starten zu lassen.

Ausgangspunkt ist die in Figur 3A dargestellte Situation, in welcher sich eine Lanzette 128 in der Applikationsposition 118 befindet. Die Pleuelstange 166 ist derart angeordnet, dass sich diese in einer oberen Position befindet, in welcher der Exzenterbolzen 210 näherungsweise in einer 12-Uhr-Position des Stechfederzahnrades 202 angeordnet ist. In dieser Stellung des Stechfederzahnrades 202 ist die Stechfeder 206 gespannt, also der mechanische Energiespeicher 204 aufgeladen (in Figur 3A nicht erkennbar, da durch das Stechfederzahnrad 202 verdeckt). Das Auslöserzahnrad 186 befindet sich in einer Position, in welcher der bezahnte Umfangsbereich 188 gerade noch in die Zähne des Stechfederzahnrades 202 eingreift. Da wiederum die Sperre 200, wie oben beschrieben, das Auslöserzahnrad 186 hält und eine Drehung desselben im Uhrzeigersinn verhindert, wird das Stechfederzahnrad 202 auf diese Weise gerade noch in der gespannten Position gehalten.

Anschließend erfolgt, ausgehend von der in Figur 3A dargestellten Position, der eigentliche Auslösevorgang. Zu diesem Zweck wird, wie durch den Pfeil 226 in Figur 3A angedeutet, dass Motorzahnrad 174 im Uhrzeigersinn gedreht. Diese Drehung 226 überträgt auf eine Drehung 228 im Gegenuhrzeigersinn des Stechantriebszahnrades 184 und, über das erste Drehrichtungssensitive Element 194, von diesem auf das Auslöserzahnrad 186. Diese Drehung 228 bewirkt jedoch, dass der bezahnte Umfangsbereich 188 des Auslöserzahnrades 186 aus den Zähnen des Stechfederzahnrades 202 ausgreift, so dass das Stechfederzahnrad 202 freigegeben wird. Nun wird dieses Stechfederzahnrad 202 nicht mehr in der gespannten Position, welche in Figur 3A dargestellt ist, gehalten, sondern entspannt sich durch eine Drehung im Gegenuhrzeigersinn. Dabei treibt die Antriebseinheit 160 über das Stechfederzahnrad 202 die Pleuelstange 166 an, welche wiederum über das Koppelstück 150 an die Lanzette 128 in der Applikationsposition 118 ankoppelt und diese in eine ausgelenkte Position antreibt. In anderen Worten wird über das Kopplungselement 152 die Lanzette 128 zu einer Stechbewegung angetrieben, welche in Figur 3A mit der Bezugsziffer 230 bezeichnet ist. Wenn der Exzenterbolzen 210 seine 9-Uhr-Position erreicht hat, erreicht die Lanzette 128 ihre stärkste Auslenkung. Anschließend bewegt sich die Pleuelstange 166 wieder zurück, da die Stechfeder 206 sich weiter entspannt. Auch das Analyseband 114 bewegt sich wieder zurück in seine nicht ausgelenkte Position. Diese Rückbewegung kann beispielsweise aktiv durch das Kopplungselement 152 erfolgen und/oder kann auf andere Weise angetrieben werden, beispielsweise durch eine Eigenspannung des Analysebandes 114. Dies macht deutlich, dass es sich in diesem Ausführungsbeispiel um eine passive Kopplung des Kopplungselementes 152 an das analytische Hilfsmittel 112 handelt. Es erfolgt jeweils ein separates Ankoppeln an jedes einzelne analytische Hilfsmittel 112 in der Applikationsposition 118, und es wird lediglich eine Vorwärtsbewegung angetrieben. Alternativ wäre jedoch auch eine aktive Kopplung möglich, bei welcher auch die Rückwärtsbewegung der Lanzette 128 bzw. des analytischen Hilfsmittels 112 angetrieben würde. Diese Stechbewegung 230 der Lanzette 128 bewirkt beispielsweise eine Perforation einer Hautpartie eines Patienten, wodurch beispielsweise ein Blutstropfen generiert wird.

Bei diesem Auslösevorgang mit einer Drehung 226 des Motorzahnrads 174 im Uhrzeigersinn wird weiterhin durch das Motorzahnrad 174 lediglich das Wickelantriebszahnrad 212 angetrieben, nicht hingegen das Transportzahnrad 216. Dies ist auf die Drehrichtungssensitivität des zweiten drehrichtungssensitiven Elements 218 zurückzuführen. Der Schlechtwickel 146 wird bei diesem Vorgang also nicht bewegt, und das Wickelantriebszahnrad 212 dreht sich leer mit, ohne den restlichen zweiten Antriebsstrang 182 zu betätigen.

In Figur 3B ist eine zweite Betriebsphase des Probengewinnungssystems 110 dargestellt, welches sich beispielsweise an die Betriebsphase in Figur 3A anschließt. Während in der Phase gemäß Figur 3A der erste Antriebsstrang 180 an das Motorzahnrad 174 gekoppelt war, um die Systemfunktion der Stechbewegung 230 zu initiieren, ist nunmehr in dem in Figur 3B dargestellten Vorgang der zweite Antriebsstrang 182 an das Motorzahnrad 174 gekoppelt, um einen Weitertransport des Analysebandes 114 zu bewirken. Während dieser Sequenz befindet sich die Stechfeder 206 in einer entspannten Position, in welcher der Exzenterbolzen 210 sich in einer 6-Uhr-Position befindet. Diese entspannte Position kann definiert gestaltet werden, indem beispielsweise ein definierter Anschlag vorgesehen wird und/oder indem die Stechfeder 206 mit einer mechanischen Vorspannung beaufschlagt wird.

Während dieser zweiten Betriebsphase wird die Drehrichtung des Motors 162 umgedreht, was beispielsweise über eine Steuerung durch ein Umpolen dieses Motors 162 erfolgen kann. Das Motorzahnrad 174 führt dementsprechend eine Drehbewegung 232 im Gegenuhrzeigersinn durch. Entsprechend dreht sich das Wickelantriebszahnrad 212 im Uhrzeigersinn, was in Figur 3B mit der Bezugsziffer 234 bezeichnet ist. Aufgrund der Drehrichtungssensitivität des zweiten drehrichtungssensitiven Elements 218 nimmt dabei das Wickelantriebszahnrad 212 das Transportzahnrad 216 mit, so dass auch dieses die Drehung 234 im Uhrzeigersinn durchführt. Das Transportzahnrad 216 greift, wie oben beschrieben, in das Wickelzahnrad 224 ein, so dass der Schlechtwickel 146 eine Drehung 236 im Gegenuhrzeigersinn durchführt. Dadurch wird das Analyseband 114 auf dem Schlechtwickel 146 aufgewickelt. Durch eine entsprechende Steuerung der Zeitdauer der Drehung bzw. des Drehwinkels des Motorzahnrades 174 (was beispielsweise wiederum über eine Steuerung des Probengewinnungssystems 110 erfolgen kann) kann hierdurch bewirkt werden, dass genau ein Testelement 122 in Form eines Testfeldes 124 in die Applikationsposition 118 gefahren wird. Vorzugsweise handelt es sich dabei um das auf die in Figur 3A verwendete Lanzette 128 nachfolgende Testelement 122 auf dem Analyseband 114. Auf diese Weise kann beispielsweise eine Taktung um genau ein analytisches Hilfsmittel 112 auf dem Analyseband 114 erfolgen.

Aufgrund der umgekehrten Drehrichtungssensitivität des ersten drehrichtungssensitiven Elementes 194 ist in dieser Sequenz der erste Antriebsstrang 180 vom Motorzahnrad 174 abgekoppelt, so dass sich beispielsweise das Stechfederzahnrad 202 und das Auslöserzahnrad 186 nicht mitdrehen. Das (in Figur 3B verdeckte) Stechantriebszahnrad 184 dreht sich zwar mit, ist jedoch von dem Auslöserzahnrad 186 über das erste drehrichtungssensitive Element 194 entkoppelt. Auf diese Weise ist sichergestellt, dass sich die Pleuelstange 166 während dieser Teilsequenz 166 nicht ändert. Das Ergebnis der in Figur 3B dargestellten Teilsequenz besteht also darin, dass sich wieder ein frisches analytisches Hilfsmittel 112 in Form eines Testelements 122 mit einem Testfeld 124 in der Applikationsposition 118 befindet. Die Stechfeder 206 ist dabei entspannt, das heißt der Energiespeicher 204 ist entladen. Bei der in Figur 3B dargestellten Teilsequenz wird also der Drehsinn des Motorzahnrades 174 im Gegenuhrzeigersinn genutzt, um über den zweiten Antriebsstrang 182 die Systemfunktion des Weitertaktens des Analysebandes 114 an den Motor 162 anzukoppeln.

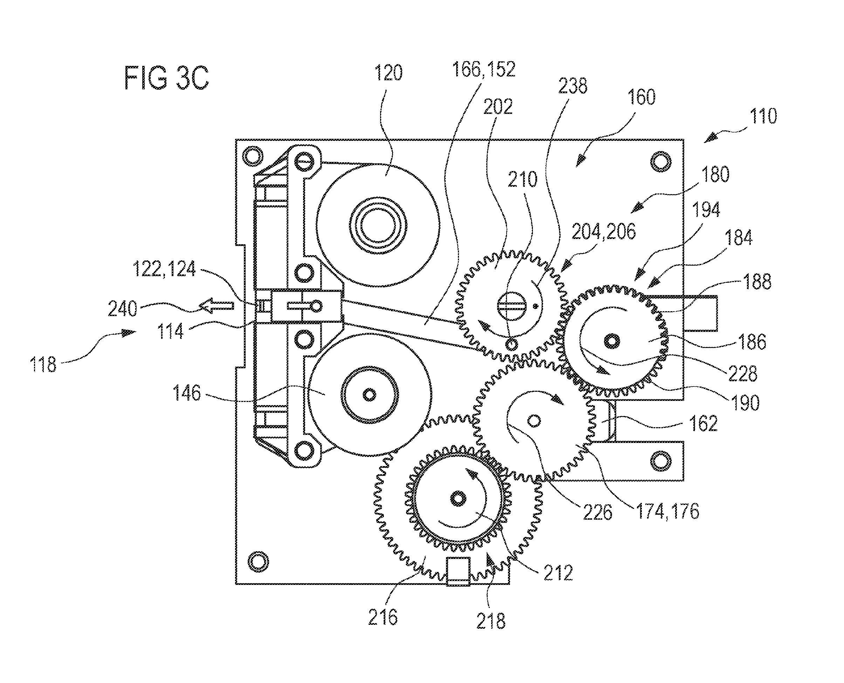

In Figur 3C ist eine dritte Teilsequenz gezeigt, bei welcher wiederum der erste Antriebsstrang 180 an den Motor 162 gekoppelt ist. Der Motor 162 wird dabei in dieser Teilsequenz derart angesteuert, dass dieser wiederum eine Drehung 226 im Uhrzeigersinn ausführt, analog zur Figur 3A. Aufgrund der Drehrichtungssensitivität des zweiten drehrichtungssensitiven Elements 218 dreht sich dabei wiederum zwar das Wickelantriebszahnrad 212 im Gegenuhrzeigersinn mit, das Transportzahnrad 216 ist von diesem jedoch durch das zweite drehrichtungssensitive Element 218 entkoppelt und steht still. Das Analyseband 114 verbleibt also in der Position, in welcher sich das Testelement 122 in der Applikationsposition 118 befindet.

Angekoppelt ist hingegen der erste Antriebsstrang 180, da das Motorzahnrad 174 das Stechantriebszahnrad 184 zu einer Drehung 228 antreibt, welches wiederum über das erste drehrichtungssensitive Element 194 das Auslöserzahnrad 186 mitnimmt. Diese Mitnahme erfolgt soweit, bis der bezahnte Umfangsbereich 188 dieses Auslöserzahnrades 186 wieder in die Zähne des Stechfederzahnrades 202 eingreift und dieses zu einer Drehung 238 im Uhrzeigersinn antreibt. Dabei laufen mehrere Vorgänge gleichzeitig ab. Zum einen wird die Stechfeder 206 wieder gespannt, so dass der mechanische Energiespeicher 204 aufgeladen wird. Gleichzeitig bewegt jedoch der Exzenterbolzen 210 die Pleuelstange 166. Dabei greift das Koppelstück 150 des Kopplungselements 152 wieder an dem Analyseband 114 an und lenkt das Testelement 122 in der Applikationsposition 118 aus. Wenn sich der Exzenterbolzen 210 in der 9-Uhr-Position befindet, so ist das Testelement 122 in der ausgelenkten Position. Anschließend, wenn sich der Exzenterbolzen 210 von der 9-Uhr-Position hin zur 12-Uhr-Position bewegt, findet eine Rückwärtsbewegung des Testelements 122 in die nicht-ausgelenkte Position statt. Diese Bewegung des Testelements 122 kann zur Probenaufnahme genutzt werden. So kann beispielsweise das Testfeld 124 eine Probennahmebewegung 240 durchführen, bei welcher das Testfeld 124 hin zu einer zuvor perforierten Hautpartie bewegt wird, dort beispielsweise einen Blutstropfen oder eine andere Art einer flüssigen Probe aufnimmt, und anschließend wieder zurückbewegt wird.

Diese Probennahmebewegung erfolgt somit zahnradgetrieben durch den Motor 174. Diese Probennahmebewegung 240 ist somit erheblich langsamer als die vorherige Auslenkung des Analysebandes 114 bei der Stechbewegung 230, was für die Probenahme vorteilhaft ist. Auf diese Weise kann sichergestellt werden, dass eine ausreichende Menge der flüssigen Probe auf das Testfeld 124 aufgebracht wird.

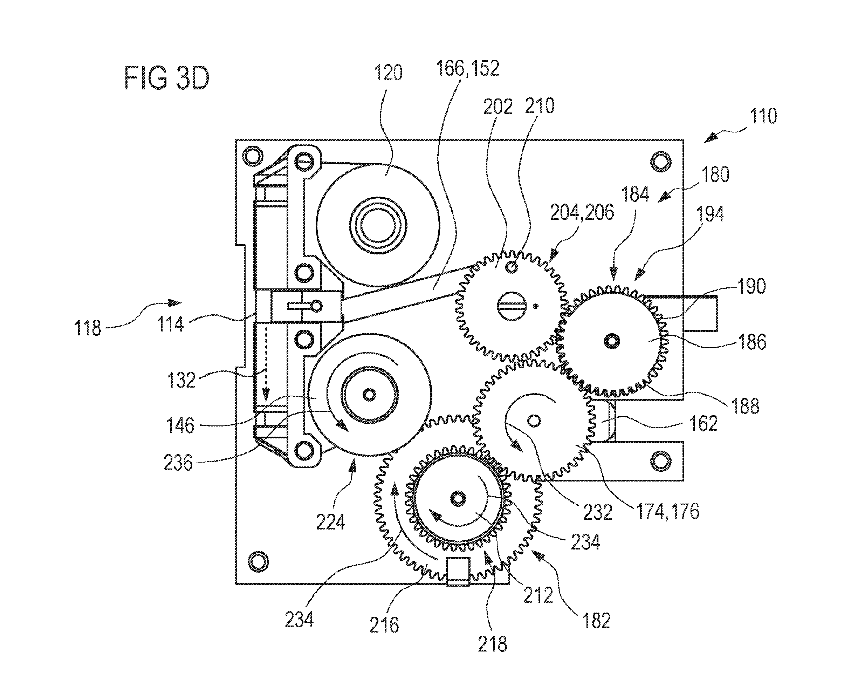

Als Resultat der in Figur 3C dargestellten Teilsequenz des Probengewinnungszyklus befindet sich somit die Stechfeder 206 wieder in der gespannten Position, analog zu Figur 3A, und eine flüssige Probe wurde auf das Testfeld 124 in der Applikationsposition 118 aufgegeben. An diese Teilsequenz kann sich eine (nicht dargestellte) Teilsequenz anschließen, bei welcher die Probe qualitativ und/oder quantitativ ausgewertet wird. Zu diesem Zweck kann beispielsweise das Testfeld 124 in der Applikationsposition 118 mit Licht beleuchtet werden, und es können durch geeignete Detektoren beispielsweise Farbänderungen einer Testchemie des Testfeldes 124 festgestellt werden, aus welchen wiederum auf eine Analytkonzentration (zum Beispiel eine Blutglukosekonzentration) in der flüssigen Probe geschlossen werden kann. Alternativ oder zusätzlich ist auch eine elektrochemische Auswertung möglich, beispielsweise indem das Analyseband 114 bzw. das Testfeld 124 elektrisch kontaktiert wird. Allgemein kann in dem dargestellten Beispiel oder auch in anderen Ausführungsbeispielen der Erfindung die Messung der Analytkonzentration jedoch vorteilhaft auch in einer anderen Position als in der Applikationsposition 118 erfolgen. So könnte beispielsweise mindestens eine separate Messposition vorgesehen sein, in welche das Testfeld 124 bewegt wird (beispielsweise im Rahmen eines nachfolgenden Verfahrensschrittes), und in welcher dieses Testfeld 124 beispielsweise optisch und/oder elektrochemisch ausgewertet wird. Dieser Transport des Testfeldes 124 in die Messposition könnte beispielsweise gleichzeitig zu dem im Folgenden anhand Figur 3D beschriebenen Transportschritt erfolgen oder könnte im Rahmen eines separaten Transportschrittes realisiert werden.

Anschließend erfolgt in Figur 3D wiederum eine Transportsequenz, bei welcher das Analyseband 114 um eine Position, das heißt um ein analytisches Hilfsmittel 112, weitergetaktet wird, so dass sich wieder eine frische Lanzette 128 in der Applikationsposition 118 befindet. Bei dieser Transport-Teilsequenz gemäß Figur 3D wird wiederum der Motor 162 derart angetrieben, dass das Motorzahnrad 174 eine Drehung 232 im Gegenuhrzeigersinn durchführt, wobei das erste drehrichtungssensitive Element 194 der erste Antriebsstrang 180 vom Motor 162 abgekoppelt ist.

Angetrieben wird jedoch wiederum, jedoch analog zu Figur 3B, das Wickelantriebszahnrad 212, durch dieses und das zweite drehrichtungssensitive Element 218 das Transportzahnrad 216, und von diesem wiederum das Wickelzahnrad 224, so dass der Schlechtwickel 146 wiederum die Drehbewegung 236 im Gegenuhrzeigersinn durchführt, durch welche das Analyseband 114 aufgewickelt wird. Auf diese Weise kann durch eine entsprechende Taktung (das heißt wiederum durch eine entsprechende Ansteuerung der Zeitdauer und/oder des Drehwinkels des Motors 162) bewirkt werden, dass sich die auf das in Figur 3C benutzte Textfeld 124 anschließende Lanzette 128 in die Applikationsposition 118 getaktet wird. Für die einzelnen Schritte dieser Teilsequenz gemäß Figur 3D kann dementsprechend auf die Beschreibung zur Figur 3B verwiesen werden. Als einziger Unterschied zur Figur 3B ist dabei zu vermerken, dass sich bei dieser Transport-Teilsequenz gemäß Figur 3D die Stechfeder 206 in einer gespannten Situation befindet, und dass der Exzenterbolzen 210 auf 12-Uhr-Position steht.

Das Ergebnis der Transport-Teilsequenz gemäß Figur 3D ist somit wiederum der Ausgangspunkt der Teilsequenz in Figur 3A. Der Zyklus der Teilsequenzen kann dementsprechend von neuem beginnen, beispielsweise um eine neue Analyse durchzuführen.

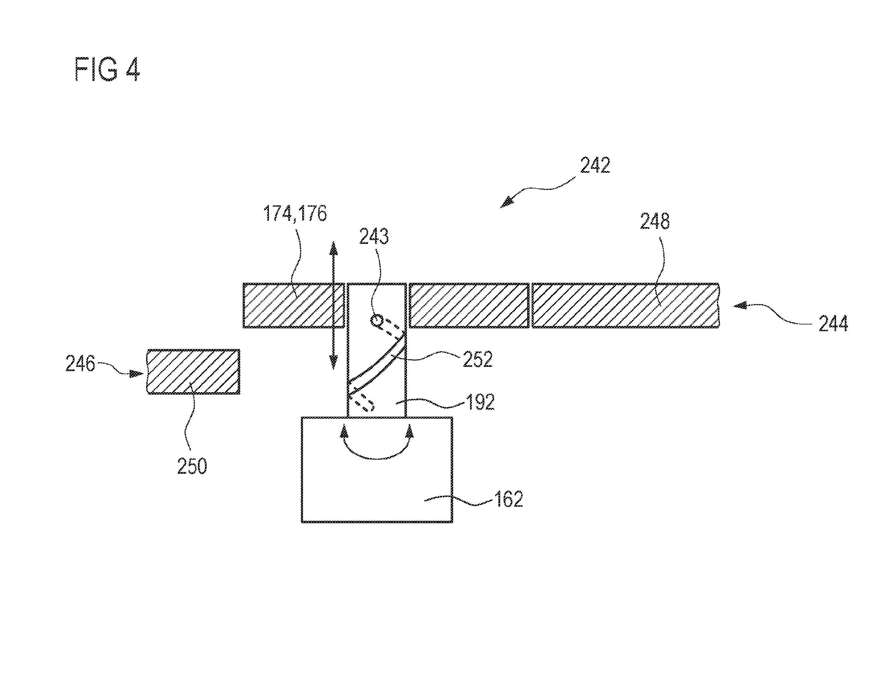

Wie oben dargestellt, ist die Verwendung der Freiläufe 196, 220 lediglich eine Möglichkeit drehrichtungssensitive Elemente 194, 218 zu realisieren. Alternativ oder zusätzlich bestehen jedoch zahlreiche weitere Möglichkeiten, eine Kopplung des Motors 162 und des Motorzahnrades 174 in unterschiedlichen Drehrichtungen an unterschiedliche Antriebsstränge 180, 182 zu realisieren, derart, dass jeweils nur ein Antriebsstrang aktiv bzw. angekoppelt ist. Eine weitere Möglichkeit einer Realisierung eines drehrichtungssensitiven Elements 242 ist exemplarisch in Figur 4 dargestellt. Wiederum wird dabei ein Motor 162 verwendet, welcher eine Motorachse 192 antreibt. Auf dieser Achse 192 sitzt wiederum ein erstes Antriebsrad 176 in Form eines Motorzahnrades 174. Dieses ist jedoch in diesem Fall derart mit der Achse 192 über einen Bolzen 243 verbunden, dass sich dieses Motorzahnrad 174 auf zwei Ebenen 244, 246 bewegen kann. Auf einer ersten Antriebsebene 244 (welche in Figur 4 oben angeordnet ist) steht dabei das Motorzahnrad 174 in Eingriff mit einem Antriebselement 248 eines ersten Antriebsstranges, wohingegen in der zweiten Antriebsebene 246 (in Figur 4 unten) das Motorzahnrad 174 in Eingriff steht mit einem Antriebselement 250 eines zweiten Antriebsstranges. Dabei müssen die beiden Antriebsstränge nicht notwendigerweise identisch sein mit den Antriebssträngen 180, 182 des Ausführungsbeispiels gemäß den Figuren 3A bis 3D, sondern können beispielsweise gegenüber diesen auch vertauscht sein.

Die Anordnung des Motorzahnrads 174 auf den zwei Ebenen 244, 246 wird dabei durch eine umfangsseitig angeordnete Nut 252 in der Achse 192 bewirkt. Der Bolzen 243 ist in dieser Nut 252 aufgenommen. Auf diese Weise wird beispielsweise bewirkt, dass durch eine Drehung der Achse 192 im Gegenuhrzeigersinn (betrachtet in Figur 4 von oben auf die Achse 192) das Motorzahnrad 174 auf die zweite Antriebsebene 146 "herunterschraubt" wird, wohingegen eine Drehung im Uhrzeigersinn der Achse 192 eine Anordnung des Motorzahnrades 174 auf der ersten Antriebsebene 244 und somit einen Eingriff in das Antriebselement 248 des ersten Antriebsstranges bewirkt. Die Nut 252 ist entsprechend einer Schraubenkurve ausgestaltet. Die Antriebselemente 248, 250 der beiden Antriebsstränge können beispielsweise wiederum als Zahnräder ausgestaltet sein. Auch andere Ausgestaltungen sind jedoch denkbar.

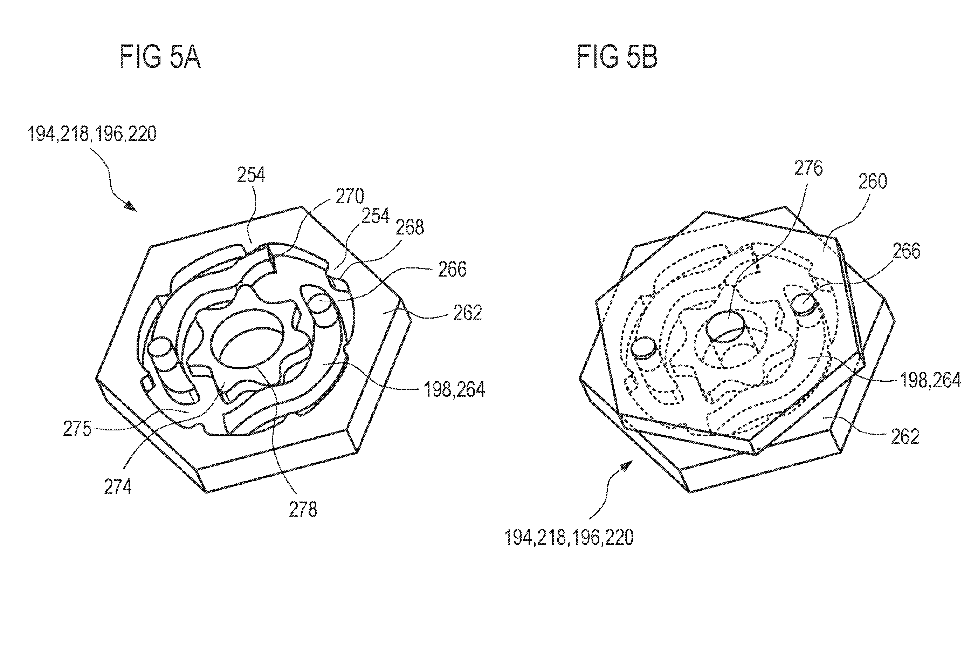

In den Figuren 5A bis 9 sind mehrere weitere Ausführungsbeispiele drehrichtungssensitiver Elemente 194, 218 in Form von Freiläufen 196, 220 in verschiedenen Darstellungen gezeigt. Dargestellt sind insbesondere Ausführungsformen von Sperrklinken-Freiläufen und eine Form eines Schlepphebel-Freilaufs. Dies schließt jedoch, wie oben beschrieben, die Verwendung anderer Arten von Freiläufen nicht aus.

Wird einer der dargestellten Freiläufe 196, 220 auf einer Antriebsseite in einer Arbeitsrichtung schneller gedreht als auf einer Abtriebsseite, so greift nach einem bauspezifischen Drehwinkel, dem Totgang, ein Mitnehmer in Form von Freilaufarmen 198 und überträgt auf die Abtriebsseite ein maximales Drehmoment. Bei Überschreitung des maximalen Drehmoments wird der Freilauf 196, 220 zerstört. Dreht sich die Antriebsseite in Gegenrichtung zur Arbeitsrichtung, so wird nur ein minimales Drehmoment, das Leerlaufdrehmoment, auf die Abtriebsseite übertragen.

Die für das vorliegende Probengewinnungssystem 110 geeigneten Freiläufe 196, 220 könnten beispielsweise Drehmomente bis ca. 100 mNm übertragen können und ein Leerlaufdrehmoment aufweisen, welches näherungsweise bei 0 mNm liegt. Zudem sollten die Freiläufe 196, 220 eine geringe Baugröße, einen einfachen Aufbau, eine robuste Konstruktion und einen geringen Totgang aufweisen und sollten leise arbeiten.

In den Figuren 5A und 5B ist ein erster Freilauf 196, 220 in verschiedenen perspektivischen Darstellungen gezeigt, welcher auf einem Freilauf in einer Automatikuhr basiert. Dieser Freilauf ist für Drehmomente von über 100 mNm konstruiert und weist ein verschwindend geringes Leerlaufdrehmoment (<1 mNm) auf.

Es handelt sich in diesem, in den Figuren 5A und 5B dargestellten Freilauf 196, 220 um ein Ausführungsbeispiel eines so genannten Schlepphebel-Freilaufs, also eines Freilaufs, bei welchem eine Antriebsseite 260 oder eine Abtriebsseite 262 (in diesem Fall die Antriebsseite 260) mit mindestens einem Freilaufarm 198 in Form eines Schlepphebels 264 verbunden ist, welcher schwenkbar um eine Achse 266 gelagert ist. Im dargestellten Ausführungsbeispiel sind zwei Schlepphebel 264 vorgesehen. Figur 2A zeigt den Freilauf 196, 220 mit abgenommener Antriebsseite 260, wohingegen in Figur 5B die Antriebsseite 260 halbtransparent dargestellt ist. Antriebsseite 260 und Abtriebsseite 262 sind dabei in diesem Ausführungsbeispiel jeweils als Sechskantscheiben ausgestaltet, wobei jedoch auch andere Ausführungsformen denkbar sind, beispielsweise Ausführungsformen in Form von Zahnscheiben.

Der Schlepphebel 264 ist asymmetrisch geformt und wirkt mit einer Zahnstruktur 254 der Abtriebsseite 262 zusammen. Bei einer Drehbewegung der Antriebsseite 260 im Gegenuhrzeigersinn gleiten die Schlepphebel 264 über flache Flanken 268 dieser Zahnstruktur 254 hinweg, ohne die Abtriebsseite 262 anzutreiben. Bei einer Drehung im Uhrzeigersinn hingegen werden Mitnahmeenden 270 der Schlepphebel 264 gegen steile Flanken 272 der Zahnstruktur 254 gedrückt und nehmen die Abtriebsseite 262 mit. Eine zweite Zahnstruktur 274 im Inneren der Abtriebsseite 262 sorgt dafür, dass sich die gekrümmten Schlepphebel 264 immer optimal an die äußere Zahnstruktur 254 anlegen. Die Zahnstruktur 254 und die zweite Zahnstruktur 274 bilden gemeinsam einen Kanal 275, in welchem die Schlepphebel 264 laufen. Im Zentrum der Antriebsseite 260 und der Abtriebsseite 262 sind jeweils Bohrungen 276, 278 vorgesehen, durch welche eine oder mehrere Achsen (nicht dargestellt) geführt und mit der Antriebsseite 260 und/oder der Abtriebsseite 262 verbunden werden können. Statt zwei Freilaufarmen 198 kann auch eine andere Anzahl von Freilaufarmen 198 vorgesehen sein, beispielsweise eine höhere Anzahl, was beispielsweise genutzt werden kann, um einen Totgang weiter zu vermindern.

Die Schlepphebelkonstruktion gemäß den Figuren 5A und 5B weist gegenüber anderen Arten von Freiläufen einige Vorteile auf, welche insbesondere für medizinische Anwendungen von Vorteil sind. Das äußerst geringe Leerlaufdrehmoment wurde bereits erwähnt.

Hierzu ist es besonders von Vorteil, wenn der Freilauf 198, 264 nicht mit einem Schmiermittel beaufschlagt werden, da derartige Schmiermittel aufgrund ihrer Zähigkeit auch in Leerlaufrichtung ein Drehmoment bewirken. Stattdessen lassen sich leichtgängige Materialien einsetzen, insbesondere Kunststoffe wie beispielsweise Polyamid und/oder Polyacetal (POM). Alternativ oder zusätzlich können auch Metallteile eingesetzt werden. Hierbei eignen sich insbesondere Metallteile, die durch einen Lasersinterprozess hergestellt sind.

Ein weiterer Grund dafür, dass das Leerlaufdrehmoment in der Schlepphebel-Konstruktion sehr gering ist, liegt darin, dass bei diesen Freiläufen in Leerlaufrichtung keine elastischen oder Verformungen der Freilaufarme 198 erforderlich sind. Die Schlepphebel 264 sind stattdessen starr ausgebildet, so dass Kräfte, welche für eine Verformung erforderlich wären, wegfallen. Gleichzeitig sind die Freilaufarme 198 in dieser Konstruktion sehr robust ausgebildet, was zu den hohen maximalen Drehmomenten von in diesem Beispiel mehr als 100 mNm führt.

Ein weiterer Vorteil der in den Figuren 5A und 5B dargestellten Konstruktion liegt in der äußerst geringen Geräuschentwicklung. Da, wie bei herkömmlichen, elastischen Freilaufarmen 198, in diesem Fall keine elastische Verformung stattfindet, welche schlagartig rückgängig gemacht würde, schlagen hier keine harten Teile aufeinander, was zu einer Geräuschentwicklung führen könnte. Die Schlepphebel 264 gleiten vielmehr über die Zahnstrukturen 254, 278, was lediglich geringe Geräusche verursacht.

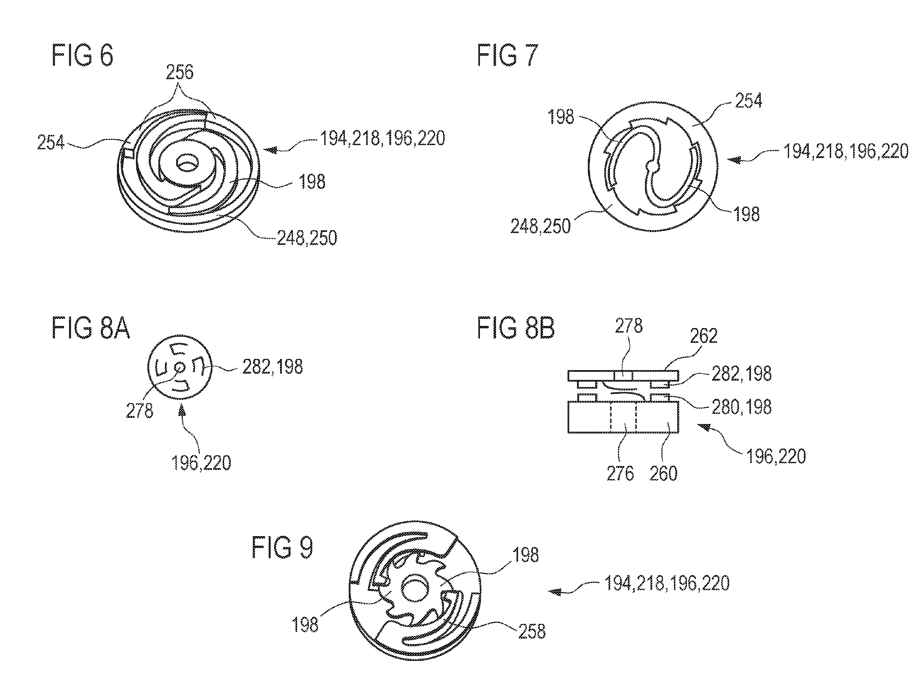

In Figur 6 ist eine Ausführungsform eines Freilaufs 196, 220 dargestellt, welcher im Wesentlichen dem Freilauf 196 in Figur 2B entspricht. Der Freilauf 196, 220 weist zwei Freilaufarme 198 auf, welche in einer Arbeitsrichtung (in Figur 6 eine Drehung der Arme zu 196 im Uhrzeigersinn) in eine Zahnstruktur 254 eines anzutreibenden Antriebselements 248, 250 eingreift. In entgegengesetzter Richtung erfolgt jedoch keine Mitnahme, da die Freilaufarme 198 über die Zahnstruktur 254 hinweggleiten. Die Zahnstruktur 254 in dem Beispiel gemäß Figur 6 weist hier zwei Zähne 256 auf. Der Freilauf gemäß Figur 6 ist für ein maximales Drehmoment von ca. 75 mNm konzipiert und weist ein Leerlaufdrehmoment von ca. 11 mNm auf.

In Figur 7 ist ein zu Figur 6 alternatives Ausführungsbeispiel eines Freilaufs 196, 220 dargestellt. Wiederum weist der Freilauf 196, 220 zwei Freilaufarme 198 auf, welche in diesem Ausführungsbeispiel ebenfalls wieder über eine Zahnstruktur 254 des anzutreibenden Antriebselements 248, 250 hinweggleiten. In diesem Ausführungsbeispiel ist die Mitnahmerichtung eine Drehrichtung der Freilaufarme 198 im Gegenuhrzeigersinn. Im Gegensatz zu Figur 6 sind zudem in dem Beispiel gemäß Figur 7 eine größere Anzahl von Zähnen 256 vorgesehen, was einen geringeren Totgang bewirkt. Der Freilauf 196, 220 gemäß Figur 7 ist konzipiert für ein maximales Drehmoment von ca. 20 mNm.

In den Figuren 8A und 8B ist eine weitere Variante eines Freilaufs 196, 220 dargestellt, welche vorzugsweise vollständig aus Stahlblech gefertigt werden kann. Hierbei sind wiederum eine Antriebsseite 260 und eine Abtriebsseite 262 vorgesehen, welche in diesem Beispiel jeweils aus kreisförmigen Stahlblechscheiben gefertigt sind. In Figur 8A ist eine Draufsicht von oben auf die Abtriebsseite 262 gezeigt, wohingegen Figur 8B den Freilauf 196, 220 in einer Seitenansicht zeigt.

Die Scheiben weisen jeweils eine Bohrung 276 bzw. 278 auf. Aus den Scheiben sind gegensinnig orientierte Freilaufarme 198 in Form von Mitnehmerhebeln 280, 282 ausgestanzt, welche die Form von Zungen aufweisen. In diesem Beispiel sind jeweils vier derartiger Mitnehmerhebel 280, 282 vorgesehen, was jedoch eine Ausführungsform mit einer unterschiedlichen Anzahl nicht ausschließt. Bei einer Drehung der Antriebsseite 260 im Gegenuhrzeigersinn in Figur 8A gleiten diese zungenförmigen Mitnehmerhebel 280, 282 übereinander hinweg, so dass keine Mitnahme erfolgt (Leerlauf). Bei einer Drehung im Uhrzeigersinn hingegen verhaken sich die Mitnehmerhebel 280, 282 ineinander, und es erfolgt eine Mitnahme.

Diese in den Figuren 8A und 8B dargestellte Variante erlaubt eine besonders flache Bauweise und ist mit zwei Mitnehmerhebeln 198 konzipiert für ein maximales Drehmoment von 50 mNm und ein Leerlaufdrehmoment von ca. 2,8 mNm. In der dargestellten Variante mit vier Freilaufarmen 198 ist der Freilauf 196, 220 für ein maximales Drehmoment von ca. 100 mNm und ein Leerlaufdrehmoment von ca. 5,6 mNm ausgelegt.

In Figur 9 ist ein weiteres Ausführungsbeispiel eines erfindungsgemäßen Freilaufs 196, 220 gezeigt. In diesem Fall sind die Freilaufarme 198 gegenüber den Freilaufarmen in den vorhergehenden Ausführungsbeispielen stark verkürzt und stellen eine Zahnradstruktur mit 8 Zähnen dar. In diese Freilaufarme 198 greifen zwei Mitnehmerarme 258 ein. Diese Mitnehmerarme bewirken, dass bei einer Drehung des Freilaufs 196, 220 im Uhrzeigersinn eine Mitnahme der Mitnehmerarme 258 erfolgt, im Gegenuhrzeigersinn hingegen nicht. Der Freilauf 196, 220 gemäß Figur 9 ist mit 2 Mitnehmerarmen 258, das heißt wie in Figur 9 dargestellt, für ein maximales Drehmoment von 25 mNm und ein Leerlaufdrehmoment von ca. 4,4 mNm konzipiert, und in einer nicht dargestellten Variante mit vier Mitnehmerarmen 258 für ein maximales Drehmoment von ca. 40 mNm und ein Leerlaufdrehmoment von ca. 6,6 mNm.

### Bezugszeichenliste

- 110: Probengewinnungssystem
- 112: Analytische Hilfsmittel
- 114: Analyseband
- 116: Grundplatte
- 118: Applikationsposition
- 120: Gutwickel
- 122: Testelemente
- 124: Testfelder
- 126: Trägerband
- 128: Lanzetten
- 130: Paar analytischer Hilfsmittel
- 132: Laufrichtung Analyseband
- 134: Markierungen
- 136: Rollensystem
- 138: feste Rollen
- 140: bewegliche Rollen
- 142: Umlenksystem
- 144: Abwickelrichtung
- 146: Schlechtwickel
- 148: Aufwickelrichtung
- 150: Koppelstück
- 152: Kopplungselement
- 154: Auslenkungsrichtung
- 156: Schlitz
- 158: Bandkassette

- 160: Antriebseinheit
- 162: Motor
- 164: Zahngetriebe
- 166: Pleuelstange
- 168: Basisplatte
- 170: Motorführung
- 172: Stützen
- 174: Motorzahnrad
- 176: erstes Antriebsrad
- 178: Motorachse
- 180: erster Antriebsstrang
- 182: zweiter Antriebsstrang
- 184: Stechantriebszahnrad
- 186: Auslöserzahnrad
- 188: bezahnter Umfangsbereich
- 190: unbezahnter Umfangsbereich
- 192: Achse
- 194: erstes drehrichtungssensitives Element
- 196: erster Freilauf
- 198: Freilaufarme
- 200: Sperre
- 201: Sperrklinke
- 202: Stechfederzahnrad
- 203: Klinkenring
- 204: mechanischer Energiespeicher
- 205: Bohrung
- 206: Stechfeder
- 208: Achse
- 210: Exzenterbolzen
- 212: Wickelantriebszahnrad
- 214: Achse
- 216: Transportzahnrad
- 218: zweites drehrichtungssensitives Element
- 220: zweiter Freilauf
- 222: Freilaufsperre
- 224: Wickelzahnrad
- 226: Drehung Motorzahnrad im Uhrzeigersinn
- 228: Drehung Stechantriebszahnrad
- 230: Stechbewegung
- 232: Drehung Motorzahnrad im Gegenuhrzeigersinn
- 234: Drehung Wickelantriebszahnrad und Transportzahnrad im Uhrzeigersinn
- 236: Drehung Schlechtwickel im Gegenuhrzeigersinn
- 238: Drehung Stechfederzahnrad im Uhrzeigersinn
- 240: Probennahmebewegung
- 242: drehrichtungssensitives Element
- 243: Bolzen
- 244: erste Antriebsebene
- 246: zweite Antriebsebene
- 248: Antriebselement erster Antriebsstrang
- 250: Antriebselement zweiter Antriebsstrang
- 252: Nut
- 254: Zahnstruktur
- 256: Zähne
- 258: Mitnehmerarme
- 260: Antriebsseite
- 262: Abtriebsseite
- 264: Schlepphebel
- 266: Achse
- 268: Flache Flanken
- 270: Mitnahmeenden
- 272: Steile Flanken
- 274: Zweite Zahnstruktur
- 275: Kanal
- 276: Bohrung
- 278: Bohrung
- 280: Mitnehmerhebel
- 282: Mitnehmerhebel

## Patentansprüche

1. Probengewinnungssystem (110) zum Gewinnen einer flüssigen Probe, aufweisend mindestens ein analytisches Hilfsmittel (112), wobei das Probengewinnungssystem (110) ein Kopplungselement (152) zur Ankopplung an das analytische Hilfsmittel (112) aufweist sowie mindestens eine Antriebseinheit (160) zum Antreiben einer Bewegung des Kopplungselements (152) aus einer Ruheposition in eine ausgelenkte Position, wobei die Antriebseinheit (160) einen Energiewandler (162) umfasst, welcher ausgestaltet ist, um eine Drehbewegung mit unterschiedlichen Drehrichtungen zu erzeugen, wobei die Antriebseinheit (160) weiterhin eine Kopplungsvorrichtung mit mindestens einem drehrichtungssensitiven Element (194, 218; 242) aufweist, wobei die Kopplungsvorrichtung eingerichtet ist, um in einer ersten Drehrichtung den Energiewandler (162) an eine erste Systemfunktion und in einer zweiten, von der ersten Drehrichtung verschiedenen Drehrichtung an eine zweite, von der ersten Systemfunktion verschiedene Systemfunktion anzukoppeln.

2. Probengewinnungssystem (110) nach dem vorhergehenden Anspruch, wobei die erste Systemfunktion und/oder die zweite Systemfunktion mindestens eine der folgenden Funktionen des Probengewinnungssystems (110) umfassen: einen Antrieb einer Stechbewegung einer Lanzette (128) des analytischen Hilfsmittels (112); eine Probennahmebewegung eines Testelements (122) des analytischen Hilfsmittels (112); ein Spannen eines Energiespeichers (204) zum Antreiben der Stechbewegung der Lanzette (128), insbesondere eines mechanischen Energiespeichers (204), insbesondere eines Federelements; eine Transportfunktion eines analytischen Hilfsmittels (112) zum Bereitstellen des analytischen Hilfsmittels (112) in einer Applikationsposition (118); eine Transportfunktion eines analytischen Hilfsmittels (112) zum Bereitstellen des analytischen Hilfsmittels (112) in einer Messposition; eine Transportfunktion eines Magazins des Probengewinnungssystems (110) zum Bereitstellen eines analytischen Hilfsmittels (112) aus einem Magazin in einer Applikationsposition (118); eine Transportfunktion eines mehrere analytische Hilfsmittel (112) enthaltenden Analysebandes (114) zum Bereitstellen eines analytischen Hilfsmittels (112) in einer Applikationsposition (118); eine Transportfunktion einer mehrere analytische Hilfsmittel (112) enthaltenden Analysescheibe zum Bereitstellen eines analytischen Hilfsmittels (112) in einer Applikationsposition (118).

3. Probengewinnungssystem (110) nach einem der beiden vorhergehenden Ansprüche, wobei das analytische Hilfsmittel (112) mindestens eine Lanzette (128) und/oder mindestens ein Testelement (122) mit einem Testfeld (124) zur Analyse der flüssigen Probe aufweist.

4. Probengewinnungssystem (110) nach dem vorhergehenden Anspruch, wobei das analytische Hilfsmittel (112) ein Analyseband (114) aufweist, wobei das Analyseband (114) eine Vielzahl von alternierend angeordneten Lanzetten (128) und Testfeldern (124) aufweist.

5. Probengewinnungssystem (110) nach einem der vorhergehenden Ansprüche, wobei das Kopplungselement (152) eingerichtet ist, um mittels einer aktiven und/oder einer passiven Kopplung an das analytische Hilfsmittel (112) anzukoppeln.

6. Probengewinnungssystem (110) nach einem der vorhergehenden Ansprüche, wobei das Kopplungselement (152) einen Pleuelantrieb (166) und/oder einen Kulissenantrieb umfasst, wobei der Pleuelantrieb (166) und/oder der Kulissenantrieb ausgestaltet sind, um an ein in einer Applikationsposition (118) angeordnetes analytisches Hilfsmittel (112) anzukoppeln.

7. Probengewinnungssystem (110) nach einem der vorhergehenden Ansprüche, wobei der Energiewandler (162) einen Elektromotor (162) umfasst.

8. Probengewinnungssystem (110) nach einem der vorhergehenden Ansprüche, umfassend einen mechanischen Energiespeicher (204), welcher eingerichtet ist, um Energie für eine Stechbewegung an eine Lanzette (128) des analytischen Hilfsmittels (112) abzugeben.

9. Probengewinnungssystem (110) nach dem vorhergehenden Anspruch, wobei der mechanische Energiespeicher (204) mindestens eines der folgenden Elemente umfasst: ein Federelement (206), insbesondere eine Schraubenfeder, eine Spiralfeder, eine Blattfeder, eine Tellerfeder; ein elastisches Element, insbesondere ein Elastomerelement; einen pneumatischen Druckspeicher.

10. Probengewinnungssystem (110) nach einem der beiden vorhergehenden Ansprüche, wobei die Antriebseinheit (160) ein Zahngetriebe (186) aufweist, wobei das Zahngetriebe (186) eingerichtet ist, um in einem ersten Winkelstellungsbereich des Energiewandlers (162) den mechanischen Energiespeicher (204) aufzuladen und in einem aufgeladenen Zustand zu halten, und wobei das Zahngetriebe eingerichtet (186) ist, um in einem zweiten Winkelstellungsbereich den mechanischen Energiespeicher (204) freizugeben, so dass Energie für die Stechbewegung der Lanzette (128) abgegeben wird.

11. Probengewinnungssystem (110) nach dem vorhergehenden Anspruch, wobei das Zahngetriebe (186) mindestens ein teilbezahntes Zahnrad (186) aufweist, welches in mindestens einem ersten Umfangsbereich (188) bezahnt ist und welches in mindestens einem zweiten Umfangsbereich (190) unbezahnt ist.

12. Probengewinnungssystem (110) nach einem der beiden vorhergehenden Ansprüche, wobei das Zahngetriebe zusätzlich eine Sperre aufweist, wobei die Sperre eingerichtet ist, um ein Entladen des Energiespeichers (204) in dem ersten Winkelstellungsbereich zu verhindern.

13. Probengewinnungssystem (110) nach einem der drei vorhergehenden Ansprüche, wobei eine Sperre (200) vorgesehen ist, um den mechanischen Energiespeicher (204) in dem aufgeladenen Zustand zu halten.

14. Probengewinnungssystem (110) nach einem der vorhergehenden Ansprüche, umfassend mindestens eines der folgenden, zur Aufnahme und Bereitstellung mehrerer analytischer Testelemente (122) geeigneten Elemente: ein Magazin, insbesondere ein Trommelmagazin, ein Stangenmagazin, ein Reihenmagazin, eine Bandkassette (158) oder ein Zickzackmagazin; ein Analyseband (114); eine Analysescheibe.

15. Probengewinnungssystem (110) nach einem der vorhergehenden Ansprüche, wobei die Antriebseinheit (160) ein Zahnradsystem umfasst, wobei das Zahnradsystem mindestens eines der folgenden Getriebeelemente umfasst: ein Stirnradgetriebe; ein Kegelradgetriebe; ein Schneckengetriebe; ein Zahnstangengetriebe; ein Spindelradgetriebe; ein Kronradgetriebe; ein Planetengetriebe.

16. Probengewinnungssystem (110) nach einem der vorhergehenden Ansprüche, wobei die Kopplungsvorrichtung ein erstes Antriebsrad (184, 212) und ein zweites Antriebselement (186, 216) umfasst, wobei das erste Antriebsrad (184, 212) mit dem Energiewandler (162) gekoppelt ist, wobei das zweite Antriebselement (186, 216) mit der ersten Systemfunktion und/oder der zweiten Systemfunktion gekoppelt ist, wobei das erste Antriebsrad (184, 212) und das zweite Antriebselement (186, 216) miteinander durch das drehrichtungssensitive Element (194, 218; 242) verbunden sind, wobei das drehrichtungssensitive Element (194, 218; 242) in der ersten Drehrichtung das erste Antriebsrad (184, 212) und das zweite Antriebselement (186, 216) miteinander koppelt und in der zweiten Drehrichtung das erste Antriebsrad (184, 212) und das zweite Antriebselement (186, 216) voneinander entkoppelt.

17. Probengewinnungssystem (110) nach dem vorhergehenden Anspruch, wobei die Kopplungsvorrichtung zwei mit dem ersten Antriebsrad (184, 212) durch zwei drehrichtungssensitive Elemente (194, 218; 242) verbundene zweite Antriebselemente (186, 216) umfasst, wobei die beiden drehrichtungssensitiven Elemente (194, 218; 242) eine entgegengesetzte Drehrichtungssensitivität aufweisen und wobei die zweiten Antriebselemente (186, 216) jeweils mit unterschiedlichen Systemfunktionen gekoppelt sind.

18. Probengewinnungssystem (110) nach einem der vorhergehenden Ansprüche, wobei das drehrichtungssensitive Element (194, 218; 242) mindestens einen Freilauf (196, 220) aufweist, insbesondere mindestens einen der folgenden Freiläufe (196, 220): einen Klemmrollenfreilauf, einen Klemmkörperfreilauf, einen Sperrklinkenfreilauf, einen Reibrichtsperrenfreilauf, einen Schlepphebelfreilauf.

19. Probengewinnungssystem (110) nach dem vorhergehenden Anspruch, wobei der Freilauf (196, 220) eine Freilaufsperre (222) zur Verringerung eines Freilauf Totgangs aufweist.

20. Probengewinnungssystem (110) nach einem der vorhergehenden Ansprüche, wobei das drehrichtungssensitive Element (194, 218; 242) ein mit dem Energiewandler (162) gekoppeltes erstes Antriebsrad (176) und mindestens zwei mit unterschiedlichen Systemfunktionen gekoppelte, in unterschiedlichen Ebenen (244, 246) angeordnete zweite Antriebselemente (248, 250) aufweist, wobei das erste Antriebsrad (176) derart eingerichtet ist, dass dieses in der ersten Drehrichtung auf einer ersten Ebene (244) angeordnet ist und an ein erstes Antriebselement (248) der zweiten Antriebselemente (248, 250) gekoppelt ist und wobei das erste Antriebsrad (176) derart eingerichtet ist, dass dieses in der zweiten Drehrichtung auf einer zweiten Ebene (246) angeordnet ist und an ein zweites Antriebselement (250) der zweiten Antriebselemente (248, 250) gekoppelt ist.

21. Probengewinnungssystem (110) nach einem der vorhergehenden Ansprüche, wobei die Kopplungsvorrichtung derart eingerichtet ist, dass der Energiewandler (162) in der ersten Drehrichtung oder der zweiten Drehrichtung gleichzeitig an eine Systemfunktion angekoppelt werden kann, in welcher ein Aufladen eines mechanischen Energiespeichers (204) mit Energie erfolgt, und an eine Systemfunktion, in welcher ein Testelement (122) in eine ausgelenkte Position überführt wird, um eine flüssige Probe auf einem Testfeld (124) des Testelements (122) zu applizieren.

22. Probengewinnungssystem (110) nach dem vorhergehenden Anspruch, wobei die Kopplungsvorrichtung derart eingerichtet ist, dass der Energiewandler (162) in der zweiten Drehrichtung an eine Systemfunktion angekoppelt werden kann, in welcher ein analytisches Hilfsmittel (112) in einer Applikationsposition (118) bereitgestellt wird.

23. Probengewinnungssystem (110) nach einem der vorhergehenden Ansprüche, wobei das Probengewinnungssystem (110) eingerichtet ist, um folgende Probennahmesequenz durchzuführen:
a) der Energiewandler (162) führt eine erste Drehbewegung in der ersten Drehrichtung aus, wobei ein mechanischer Energiespeicher (204) freigegeben wird und Energie für eine Stechbewegung einer in einer Applikationsposition (118) angeordneten Lanzette (128) des analytischen Hilfsmittels (112) abgibt;
b) der Energiewandler (162) führt eine zweite Drehbewegung in der zweiten Drehrichtung aus, wobei ein Testelement (122) mit einem Testfeld (124) zur Analyse der flüssigen Probe in die Applikationsposition (118) überführt wird;
c) der Energiewandler (162) führt eine dritte Drehbewegung in der ersten Drehrichtung aus, wobei ein Aufladen des mechanischen Energiespeichers (204) mit Energie erfolgt und wobei das Testelement (122) in eine ausgelenkte Position überführt wird, um eine flüssige Probe auf dem Testfeld (124) des Testelements (122) zu applizieren;
d) der Energiewandler (162) führt eine vierte Drehbewegung in der zweiten Drehrichtung aus, wobei eine Lanzette (128) in die Applikationsposition (118) überführt wird.

24. Bandkassette (158) zum Einsatz in einem Probengewinnungssystem (110) nach einem der vorhergehenden Ansprüche, umfassend ein Analyseband (114), einen Gutwickel (120) und einen Schlechtwickel (146) zur Aufnahme des Analysebandes (114) sowie ein Koppelstück (150), wobei das Koppelstück (150) eingerichtet ist, um als Teil des Kopplungselements (152) des Probengewinnungssystems (110) zu wirken.

25. Verfahren zum Gewinnen einer flüssigen Probe, wobei ein Probengewinnungssystem (110) verwendet wird, welches mindestens ein analytisches Hilfsmittel (112) aufweist, wobei das Probengewinnungssystem (110) weiterhin ein Kopplungselement (152) zur Ankopplung an das analytische Hilfsmittel (112) aufweist sowie mindestens eine Antriebseinheit (160) zum Antreiben einer Bewegung des Kopplungselements (152) aus einer Ruheposition in eine ausgelenkte Position, wobei die Antriebseinheit (160) einen Energiewandler (162) umfasst, welcher ausgestaltet ist, um eine Drehbewegung mit unterschiedlichen Drehrichtungen zu erzeugen, wobei die Antriebseinheit (160) weiterhin eine Kopplungsvorrichtung mit mindestens einem drehrichtungssensitiven Element (194, 218; 242) aufweist, wobei die Kopplungsvorrichtung eingerichtet ist, um in einer ersten Drehrichtung den Energiewandler (162) an eine erste Systemfunktion und in einer zweiten, von der ersten Drehrichtung verschiedenen Drehrichtung an eine zweite, von der ersten Systemfunktion verschiedene Systemfunktion anzukoppeln, wobei das Verfahren folgende Schritte umfasst:
a) der Energiewandler (162) führt eine erste Drehbewegung in der ersten Drehrichtung aus, wobei ein mechanischer Energiespeicher (204) freigegeben wird und Energie für eine Stechbewegung einer in einer Applikationsposition (118) angeordneten Lanzette (128) des analytischen Hilfsmittels (112) abgibt;
b) der Energiewandler (162) führt eine zweite Drehbewegung in der zweiten Drehrichtung aus, wobei ein Testelement (122) mit einem Testfeld (124) zur Analyse der flüssigen Probe in die Applikationsposition (118) überführt wird;
c) der Energiewandler (162) führt eine dritte Drehbewegung in der ersten Drehrichtung aus, wobei ein Aufladen des mechanischen Energiespeichers (204) mit Energie erfolgt und wobei das Testelement (122) in eine ausgelenkte Position überführt wird, um eine flüssige Probe auf dem Testfeld (124) des Testelements (122) zu applizieren;
d) der Energiewandler (162) führt eine vierte Drehbewegung in der zweiten Drehrichtung aus, wobei eine Lanzette (128) in die Applikationsposition (118) überführt wird.
